# EUROPEAN PATENT SPECIFICATION

(11) **EP 3 864 657 B1**
(45) Date of publication and mention of the grant of the patent: **04.12.2024**
(21) Application number: 19870462.9
(22) Date of filing: 12.10.2019
(51) Int. Cl.: G16B 40/20, G16B 40/30, G16B 20/40

(54) **ENRICHMENT OF TRAITS AND ASSOCIATION WITH POPULATION DEMOGRAPHY**
ANREICHERUNG VON MERKMALEN UND ASSOZIATION MIT POPULATIONSDEMOGRAPHIE
ENRICHISSEMENT DE TRAITS ET ASSOCIATION AVEC LA DÉMOGRAPHIE D'UNE POPULATION

(30) Priority: 12.10.2018 US 201862745245 P; 17.10.2018 US 201862747034 P; 03.05.2019 US 201962843164 P
(43) Date of publication of application: 18.08.2021
(73) Proprietor: Ancestry.com DNA, LLC, Lehi, UT 84043 (US)
(72) Inventor: TELIS, Natalie, Lehi, Utah 84043 (US); SOROKIN, Elena P., Lehi, Utah 84043 (US); RAND, Kristin A., Lehi, Utah 84043 (US); GRANKA, Julie M., Lehi, Utah 84043 (US)
(74) Representative: Boult Wade Tennant LLP
(86) International application number: PCT/IB2019/058717
(87) International publication number: WO 2020/075145

(56) References cited:
- US-A1- 2004 267 458
- US-A1- 2010 256 917
- US-A1- 2014 067 355
- US-A1- 2016 350 479
- US-A1- 2017 017 752
- US-A1- 2017 213 127
- ULIVI SHEILA ET AL: "Genetics of eye colours in different rural populations on the Silk Road", EUROPEAN JOURNAL OF HUMAN GENETICS, vol. 21, no. 11, 13 March 2013 (2013-03-13), CH, pages 1320 - 1323, XP055922275, ISSN: 1018-4813, Retrieved from the Internet <URL:https://www.ncbi.nlm.nih.gov/pmc/articles/PMC3798846/pdf/ejhg201341a.pdf> [retrieved on 20220517], DOI: 10.1038/ejhg.2013.41
- ZHU, X. ET AL.: "A large-scale genome-wide enrichment analysis identifies new trait-associated genes, pathways and tissues across 31 human phenotypes", BIORXIV, 7 July 2017 (2017-07-07), pages 1 - 30, XP055703950, Retrieved from the Internet <URL:https://www.biorxiv.org/content/10.1101/160770v2.full.pdf÷html>
- HILL, W. D. ET AL.: "A combined analysis of genetically correlated traits identifies 187 loci and a role for neurogenesis and myelination in intelligence", MOLECULAR PSYCHIATRY, vol. 24, 2018, pages 169 - 181, XP036695876, DOI: 10.1038/s41380-017-0001-5
- LEE, S. ET AL.: "Rare-variant association analysis: study designs and statistical tests", THE AMERICAN JOURNAL OF HUMAN GENETICS, vol. 95, 3 July 2014 (2014-07-03), pages 5 - 23, XP055625895, DOI: 10.1016/j.ajhg.2014.06.009

## Description

### BACKGROUND

Although humans are, genetically speaking, almost entirely identical, small differences in human DNA are responsible for some observed variations between individuals. The human genome mutation rate is estimated to be 1.1*10^-8 per site per generation. This leads to a variant approximately every 300 base pairs. Most of the mutations that are passed down to descendants are related to single-nucleotide polymorphism (SNP). SNP is a substitution of a single nucleotide that occurs at a specific position in the genome. Learning about population structure from genetic polymorphism data is an important topic in genetics.

ULIVI SHEILA ET AL: "Genetics of eye colours in different rural populations on the Silk Road", EUROPEAN JOURNAL OF HUMAN GENETICS, vol. 21, no. 11, 1 November 2013 (2013-11-01), pages 1320-1323, describes genetics of eye colours in different rural populations on the silk road. ULIVI SHEILA ET AL does not disclose identifying genetic communities based on SNP values and performing an association study for a phenotype within genetic communities in which the phenotype was found to be enriched.

US2016/350479 A1 describes techniques for determining population structure from identity-by-descent (IBD) of individuals. US2016/350479 A1 does not disclose performing an association study related of a phenotype within the genetic communities or predicting an individual's phenotype from SNP values.

### SUMMARY OF THE INVENTION

The invention is set out in the appended claims.

### BRIEF DESCRIPTION OF THE DRAWINGS

Figure (FIG.) 1 illustrates a diagram of a system environment of an example computing system, in accordance with an embodiment.
FIG. 2 is a block diagram of an architecture of an example computing system, in accordance with an embodiment.
FIG. 3 is a flowchart illustrating an example process to analyze a trait from profile metadata of an individual, in accordance with an embodiment.
FIGS. 4A, 4B, and 4C are example geographical maps showing locations of birth of individuals whose grandparents speak a particular native language.
FIG. 5 is an example plot of the genetic ancestry composition of individuals with the composite trait that at least one of the grandparent of an individual speaks the same native language with the individual.
FIG. 6 is an example plot that illustrates the heritability of languages for individuals having the composite trait that two of their grandparents speak one language.
FIGS. 7A, 7B, 7C are example plots illustrating potential sex-bias in admixture individuals with composite traits that are defined based on maternal grandparent languages and paternal grandparent languages.
FIG. 8 is a flowchart illustrating an example process to analyze a trait from profile metadata of an individual, in accordance with an embodiment.
FIG. 9 is an example plot showing the preference of sweetened carbonated beverages for members of different genetic communities.
FIG. 10 is an example plot showing the log odds ratios of male-pattern baldness for different genetic communities.
FIG. 11A and 11B are example plots showing the log odds ratios of brown eyes versus blue eyes for different genetic communities.
FIG. 12 is an example plot of a result of an association study of male-pattern baldness of the genetic community of a cohort of individuals of European ancestry.
FIG. 13 is a block diagram of an example computing device, in accordance with an embodiment.

The figures depict various embodiments for purposes of illustration only. One skilled in the art will readily recognize from the following discussion that alternative embodiments of the structures and methods illustrated herein may be employed without departing from the principles described herein.

### SUMMARY OF RELATED DISCLOSURE

The figures (FIGs.) and the following description relate to preferred embodiments by way of illustration only. One of skill in the art may recognize alternative embodiments of the structures and methods disclosed herein as viable alternatives that may be employed without departing from the principles of what is disclosed.

Reference will now be made in detail to several embodiments, examples of which are illustrated in the accompanying figures. It is noted that wherever practicable similar or like reference numbers may be used in the figures and may indicate similar or like functionality. The figures depict embodiments of the disclosed system (or method) for purposes of illustration only. One skilled in the art will readily recognize from the following description that alternative embodiments of the structures and methods illustrated herein may be employed without departing from the principles described herein.

### EXAMPLE SYSTEM ENVIRONMENT

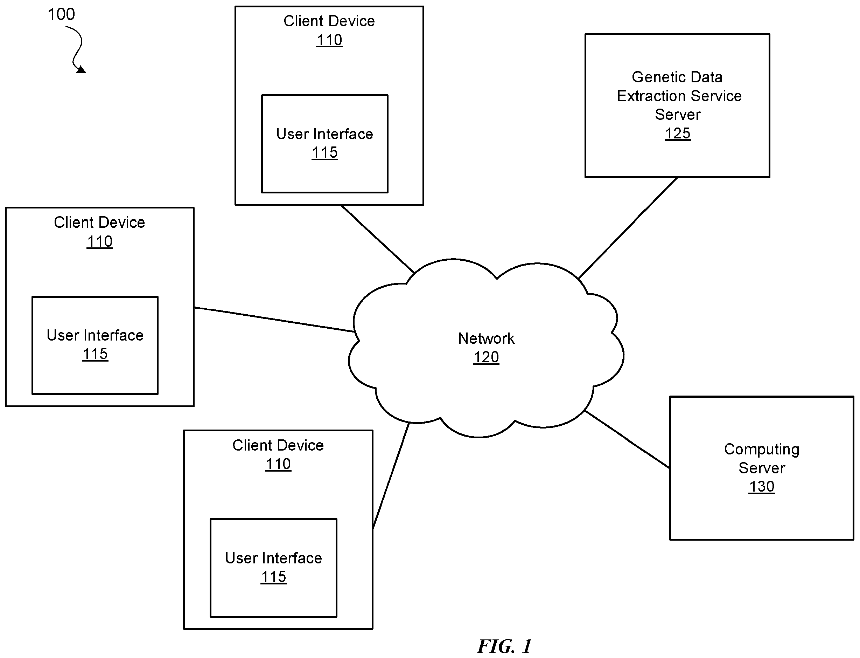

FIG. 1 illustrates a diagram of a system environment 100 of an example computing server 130, in accordance with an embodiment. The system environment 100 shown in FIG. 1 includes one or more client devices 110, a network 120, a genetic data extraction service server 125, and a computing server 130. In various embodiments, the system environment 100 may include fewer or additional components. The system environment 100 may also include different components.

The client devices 110 are one or more computing devices capable of receiving user input as well as transmitting and/or receiving data via a network 120. Example computing devices include desktop computers, laptop computers, personal digital assistants (PDAs), smartphones, tablets, wearable electronic devices (e.g., smartwatches), smart household appliance (e.g., smart televisions, smart speakers, smart home hubs), Internet of Things (IoT) devices or other suitable electronic devices. A client device 110 communicates to other components via the network 120. Users may be customers of the computing server 130 or any individuals who access the system of the computing server 130, such as an online website or a mobile application. In one embodiment, a client device 110 executes an application that launches a graphical user interface (GUI) for a user of the client device 110 to interact with the computing server 130. The GUI may be an example of a user interface 115. A client device 110 may also execute a web browser application to enable interactions between the client device 110 and the computing server 130 via the network 120. In another embodiment, the user interface 115 may take the form of a software application published by the computing server 130 and installed on the user device 110. In yet another embodiment, a client device 110 interacts with the computing server 130 through an application programming interface (API) running on a native operating system of the client device 110, such as IOS or ANDROID.

The network 120 provides connections to the components of the system environment 100 through one or more sub-networks, which may include any combination of local area and/or wide area networks, using both wired and/or wireless communication systems. In one embodiment, a network 120 uses standard communications technologies and/or protocols. For example, a network 120 may include communication links using technologies such as Ethernet, 802.11, worldwide interoperability for microwave access (WiMAX), 3G, 4G, Long Term Evolution (LTE), 5G, code division multiple access (CDMA), digital subscriber line (DSL), etc. Examples of network protocols used for communicating via the network 120 include multiprotocol label switching (MPLS), transmission control protocol/Internet protocol (TCP/IP), hypertext transport protocol (HTTP), simple mail transfer protocol (SMTP), and file transfer protocol (FTP). Data exchanged over a network 120 may be represented using any suitable format, such as hypertext markup language (HTML) or extensible markup language (XML). In some embodiments, all or some of the communication links of a network 120 may be encrypted using any suitable technique or techniques such as secure sockets layer (SSL), transport layer security (TLS), virtual private networks (VPNs), Internet Protocol security (IPsec), etc. The network 120 also includes links and packet switching networks such as the Internet.

Individuals, who may be customers of a company operating the computing server 130, provide biological samples for analysis of their genetic data. Individuals may also be referred to as users. In one embodiment, an individual uses a sample collection kit to provide a biological sample (e.g., saliva, blood, hair, tissue) from which genetic data is extracted and determined according to nucleotide processing techniques such as amplification and sequencing. Amplification may include using polymerase chain reaction (PCR) to amplify segments of nucleotide samples. Sequencing may include sequencing of deoxyribonucleic acid (DNA) sequencing, ribonucleic acid (RNA) sequencing, etc. Suitable sequencing techniques may include Sanger sequencing and massively parallel sequencing such as various next-generation sequencing (NGS) techniques including whole genome sequencing, pyrosequencing, sequencing by synthesis, sequencing by ligation, and ion semiconductor sequencing. Genetic data extraction service server 125 receives biological samples from users of the computing server 130. The genetic data extraction service server 125 performs sequencing of the biological samples and determines the base pair sequences of the individuals. The genetic data extraction service server 125 generates the genetic data of the individuals based on the sequencing results. The genetic data may include data sequenced from DNA or RNA and may include base pairs from coding and/or noncoding regions of DNA.

The genetic data may take different forms. For example, in one embodiment, the genetic data may be the base pair sequence of an individual. The base pair sequence may include the whole genome or a part of the genome such as certain genetic loci of interest. In another embodiment, the genetic data extraction service server 125 may determine genotypes from sequencing results, for example by identifying genotype values of single nucleotide polymorphisms (SNPs) present within the DNA. The results in this example may include a sequence of genotypes corresponding to various SNP sites. A SNP site may also be referred to as a SNP locus. A genetic locus is a segment of a genetic sequence. A locus can be a single site or a longer stretch. The segment can be a single base long or multiple bases long. In one embodiment, the genetic data extraction service server 125 may perform data pre-processing of the genetic data to convert raw sequences of base pairs to sequences of genotypes at target SNP sites. Since a typical human genome may differ from a reference human genome at only several million SNP sites (as opposed to billions of base pairs in the whole genome), the genetic data extraction service server 125 may extract only the genotypes at a set of target SNP sites and transmit the extracted data to the computing server 130 as the genetic dataset of an individual.

The computing server 130 performs various analyses of the genetic data, genealogical data, and users' survey responses to generate results regarding the phenotypes and genealogy of users of computing server 130. Depending on the embodiments, the computing server 130 may also be referring to as an online server, a personal genetic service server, a genealogy server, a family tree building server, and/or a social networking system. The computing server 130 receives genetic data from the genetic data extraction service server 125 and stores the genetic data in the data store of the computing server 130. The computing server 130 may analyze the data to generate results regarding the genetics or genealogy of users. The results regarding the genetics or genealogy of users may include the ethnicity compositions of users, paternal and maternal genetic analysis, identification or suggestion of potential family relatives, ancestor information, analyses of DNA data, potential or identified traits such as phenotypes of users (e.g., diseases, appearance traits, other genetic characteristics, and other non-genetic characteristics including social characteristics), etc. The computing server 130 may present or cause the user interface 115 to present the results to the users through a GUI displayed at the client device 110. The results may include graphical elements, textual information, data, charts, and other elements such as family trees.

In one embodiment, the computing server 130 also allows various users to create one or more genealogical profiles of the user. The genealogical profile may include a list of individuals (e.g., ancestors, relatives, friends, and other people of interest) who are added or selected by the user or suggested by the computing server 130 based on the genealogical records and/or genetic records. The user interface 115 controlled by or in communication with the computing server 130 may display the individuals in a list or as a family tree such as in the form of a pedigree chart. In one embodiment, subject to user's privacy setting and authorization, the computing server 130 may allow information generated from the user's genetic dataset to be linked to the user profile and to one or more of the family trees. The users may also authorize the computing server 130 to analyze their genetic dataset and allow their profiles to be discovered by other users.

### EXAMPLE COMPUTING SERVER ARCHITECTURE

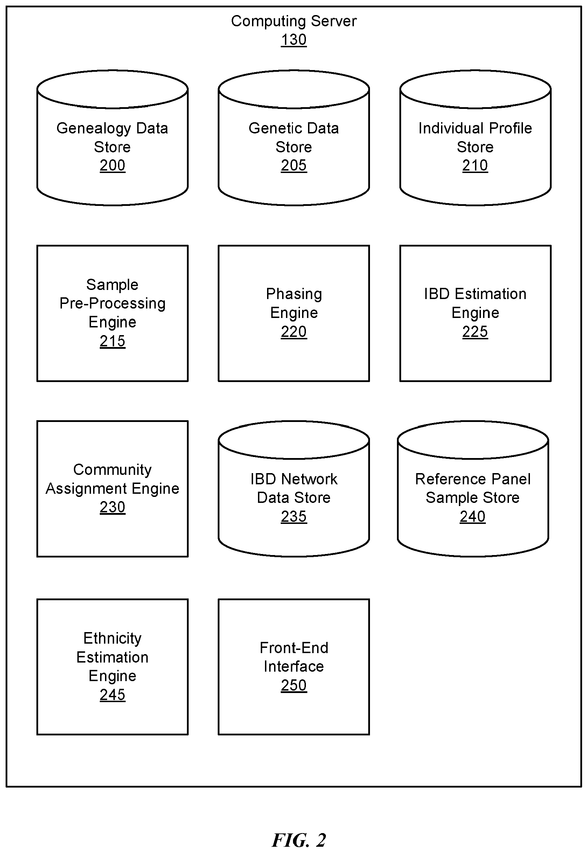

FIG. 2 is a block diagram of an architecture of an example computing server 130, in accordance with an embodiment. In the embodiment shown in FIG. 2, the computing server 130 includes a genealogy data store 200, a genetic data store 205, a individual profile store 210, a sample pre-processing engine 215, a phasing engine 220, an identity by descent (IBD) estimation engine 225, a community assignment engine 230, an IBD network data store 235, a reference panel sample store 240, an ethnicity estimation engine 245, and a front-end interface 250. The functions of the computing server 130 may be distributed among the elements in a different manner than described. In various embodiments, the computing server 130 may include different components and fewer or additional components. Each of the various data stores may be a single storage device, a server controlling multiple storage devices, or a distributed network that is accessible through multiple nodes (e.g., a cloud storage system).

The computing server 130 stores various data of different individuals, including genetic data, genealogical data, and survey response data. The computing server 130 processes the genetic data of users to identify shared identity-by-descent (IBD) segments between individuals. The genealogical data and survey response data may be part of user profile data. The amount and type of user profile data stored for each user may vary based on the information of a user, which is provided by the user as she creates an account and profile at a system operated by the computing server 130 and continues to build her profile, family tree, and social network at the system and to link her profile with her genetic data. Users may provide data via the user interface 115 of a client device 110. Initially and as a user continues to build her genealogical profile, the user may be prompted to answer questions related to basic information of the user (e.g., name, date of birth, birthplace, etc.) and later on more advanced questions that may be useful for obtaining additional genealogical data. The computing server 130 may also include survey questions regarding various traits of the users such as the users' phenotypes, characteristics, preferences, habits, lifestyle, environment, etc.

Genealogical data may be stored in the genealogical data store 200 and may include various types of data that are related to tracing family relatives of users. Examples of genealogical data include names (first, last, middle, suffixes), gender, birth locations, date of birth, date of death, marriage information, spouse's information kinships, family history, dates and places for life events (e.g., birth and death), other vital data, and the like. In some instances, family history can take the form of a pedigree of an individual (e.g., the recorded relationships in the family). The family tree information associated with an individual may include one or more specified nodes. Each node in the family tree represents the individual, an ancestor of the individual who might have passed down genetic material to the individual, and the individual's other relatives including siblings, cousins, offspring in some cases. Genealogical data may also include connections and relationships among users of the computing server 130. The information related to the connections among a user and her relatives that may be associated with a family tree may also be referred to as pedigree data or family tree data.

In addition to user-input data, genealogical data may also take other forms that are obtained from various sources such as public records and third-party data collectors. For example, genealogical records from public sources include birth records, marriage records, death records, census records, court records, probate records, adoption records, obituary records, etc. Likewise, genealogical data may include data from one or more of a pedigree of an individual, the Ancestry World Tree system, a Social Security Death Index database, the World Family Tree system, a birth certificate database, a death certificate database, a marriage certificate database, an adoption database, a draft registration database, a veterans database, a military database, a property records database, a census database, a voter registration database, a phone database, an address database, a newspaper database, an immigration database, a family history records database, a local history records database, a business registration database, a motor vehicle database, and the like.

Furthermore, the genealogical data store 200 may also include relationship information inferred from the genetic data stored in the genetic data store 205 and information received from the individuals. For example, the relationship information may indicate which individuals are genetically related, how they are related, how many generations back they share common ancestors, lengths and locations of IBD segments shared, which genetic communities an individual is a part of, variants carried by the individual, and the like.

The computing server 130 maintains genetic datasets of individuals in the genetic data store 205. A genetic dataset of an individual may be a digital dataset of nucleotide data (e.g., SNP data) and corresponding metadata. A genetic dataset may contain data of the whole or portions of an individual's genome. The genetic data store 205 may store a pointer to a location associated with the genealogical data store 200 associated with the individual. A genetic dataset may take different forms. In one embodiment, a genetic dataset may take the form of a base pair sequence of the sequencing result of an individual. A base pair sequence dataset may include the whole genome of the individual (e.g., obtained from a whole-genome sequencing) or some parts of the genome (e.g., genetic loci of interest).

In another embodiment, a genetic dataset may take the form of sequences of genetic markers. Examples of genetic markers may include target SNP loci (e.g., allele sites) filtered from the sequencing results. A SNP locus that is single base pair long may also be referred to a SNP site. A SNP locus may be associated with a unique identifier. The genetic dataset may be in a form of a diploid data that includes a sequencing of genotypes, such as genotypes at the target SNP loci, or the whole base pair sequence that includes genotypes at known SNP loci and other base pair sites that are not commonly associated with known SNPs. The diploid dataset may be referred to as a genotype dataset or a genotype sequence. Genotype may have a different meaning in various contexts. In one context, an individual's genotype may refer to a collection of diploid alleles of an individual. In other contexts, a genotype may be a pair of alleles present on two chromosomes for an individual at a given genetic marker such as a SNP site.

A genotype at a SNP site may include a pair of alleles. The pair of alleles may be homozygous (e.g., A-A or G-G) or heterozygous (e.g., A-T, C-T). Instead of storing the actual nucleotides, the genetic data store 205 may store genetic data that are converted to bits. For a given SNP site, oftentimes only two nucleotide alleles (instead of all 4) are observed. As such, a 2-bit number may represent a SNP site. For example, 00 may represent homozygous first alleles, 11 may represent homozygous second alleles, and 01 or 10 may represent heterozygous alleles. A separate library may store what nucleotide corresponds to the first allele and what nucleotide corresponds to the second allele at a given SNP site.

A diploid dataset may also be phased into two sets of haploid data, one corresponding to a first parent side and another corresponding to a second parent side. The phased datasets may be referred to as haplotype datasets or haplotype sequences. Similar to genotype, haplotype may have a different meaning in various contexts. In one context, a haplotype may also refer to a collection of alleles that corresponds to a genetic segment. In other contexts, a haplotype may refer to a specific allele at a SNP site. For example, a sequence of haplotypes may refer to a sequence of alleles of an individual that are inherited from a parent.

The individual profile store 210 stores profiles and related metadata associated with various individuals appeared in the computing server 130. A computing server 130 may use unique individual identifiers to identify various users and other non-users that might appear in other data sources such as ancestors or historical persons who appear in any family tree or genealogical database. A unique individual identifier may a hash of certain identification information of an individual, such as a user's account name, user's name, date of birth, location of birth, or any suitable combination of the information. The profile data related to an individual may be stored as metadata associated with an individual's profile. For example, the unique individual identifier and the metadata may be stored as a key-value pair using the unique individual identifier as a key.

An individual's profile data may include various kinds of information related to the individual. The metadata about the individual may include one or more pointer associating genetic datasets such as genotype and phased haplotype data of the individual that are saved in the genetic data store 205. The metadata about the individual may also individual information related to family trees and pedigree datasets that include the individual. The profile data may further include declarative information about the user that was authorized by the user to be shared and may also include information inferred by the computing server 130. Other examples of information stored in a user profile may include biographic, demographic, and other types of descriptive information such as work experience, educational history, gender, hobbies, or preferences, location and the like. In one embodiment, the user profile data may also include one or more photos of the users and photos of relatives (e.g., ancestors) of the users that are uploaded by the users. A user may authorize the computing server 130 to analyze one or more photos to extract information, such as user's or relative's appearance traits (e.g., blue eyes, curved hair, etc.), from the photos. The appearance traits and other information extracted from the photos may also be saved in the profile store. User profile data may also be obtained from other suitable sources, including historical records (e.g., records related to an ancestor), medical records, military records, photographs, other records indicating one or more traits, and other suitable recorded data.

For example, the computing server 130 may present various survey questions to its users from time to time. The responses to the survey questions may be stored at individual profile store 210. The survey questions may be related to various aspects of the users and the users' families. Some survey questions may be related to users' phenotypes, while other quesetions may be related to environmental factors of the users.

Survey questions may concern health or disease-related phenotypes, such as questions related to the presence or absence of genetic diseases or disorders, inheritable diseases or disorders, or other common diseases or disorders that have family history as one of the risk factors, questions regarding any diagnosis of increased risk of any diseases or disorders, and questions concerning wellness-related issues such as family history of obesity, family history of causes of death, etc. The diseases identified by the survey questions may be related to single-gene diseases or disorders that are caused by a single-nucleotide variant, an insertion, or a deletion. The diseases identified by the survey questions may also be multifactorial inheritance disorders that may be caused by a combination of environmental factors and genes. Examples of multifactorial inheritance disorders may include heart disease, Alzheimer's diseases, diabetes, cancer, and obesity. The computing server 130 may obtain data of a user's disease-related phenotypes from survey questions of health history of the user and her family and also from health records uploaded by the user.

Survey questions also may be related to other types of phenotypes such as appearance traits of the users. A survey regarding appearance traits and characteristics may include questions related to eye color, iris pattern, freckles, chin types, finger length, dimple chin, earlobe types, hair color, hair curl, skin pigmentation, susceptibility to skin burn, bitter taste, male baldness, baldness pattern, presence of unibrow, presence of wisdom teeth, height, and weight. A survey regarding other traits also may include questions related to users' taste and smell such as the ability to taste bitterness, asparagus smell, cilantro aversion, etc. A survey regarding traits may further include questions related to users' body conditions such as lactose tolerance, caffeine consumption, malaria resistance, norovirus resistance, muscle performance, alcohol flush, etc. Other survey questions regarding a person's physiological or psychological traits may include vitamin traits and sensory traits such as ability to sense an asparagus metabolite. Traits may also be collected from historical records, electronic health records and electronic medical records.

The computing server 130 also may present various survey questions related to environmental factors of users. In this context, an environmental factor may be a factor that is not directly connected to the genetics of the users. Environmental factors may include users' preferences, habits, and lifestyle. For example, a survey regarding users' preferences may include questions related to things and activities that users like or dislike, such as types of music a user enjoys, dancing preference, party-going preference, certain sports that a user plays, video games preferences, etc. Other questions may be related to the users' diet preference such as like or dislike a certain type of food (e.g., ice cream, egg). A survey related to habits and lifestyle may include questions regarding smoking habits, alcohol consumption and frequency, daily exercise duration, sleeping habits (e.g., morning person versus night person), sleeping cycles and problems, hobbies, and travel preferences. Additional environmental factors may include diet amount (calories, macronutrients), physical fitness abilities (e.g. stretching, flexibility, heart rate recovery), family type (adopted family or not, has siblings or not, lived with extended family during childhood), property and item ownership (has home or rents, has smartphone or doesn't, has car or doesn't).

Surveys also may be related to other environmental factors such as geographical, social-economic, or cultural factors. Geographical questions may include questions related to the birth location, family migration history, town or city of users' current or past residence. Social-economic questions may be related to users' education level, income, occupations, self-identified demographic groups, etc. Questions related to culture may concern users' religions, native language, language spoken at home, customs, dietary practices, etc. Other questions related to users' cultural and behavioral questions are also possible. Questions may also ask users' beliefs or opinions such as political beliefs, religious beliefs, opinions on certain debates, events, and controversies, and opinions on any suitable things or concepts.

For any survey questions asked, the computing server 130 may also ask an individual the same or similar questions regarding the traits of the ancestors, family members, other relatives or friends of the individual. For example, a user may be asked about the native language of the user and the native languages of the user's parents and grandparents. A user may also be asked about the health history of his or her family members.

In addition to storing the survey data in the individual profile store 210, the computing server 130 may store some responses that correspond to data related to genealogical and genetics respectively to genealogical data store 200 and genetic data store 205.

The user profile data, survey response data, the genetic data, and the genealogical data may subject to the privacy and authorization setting from the users. For example, when presented with a survey question, a user may select to answer or skip the question. The computing server 130 may present users from time to time information regarding users' selection of the extent of information and data shared. The computing server 130 also may maintain and enforce one or more privacy settings for users in connection with the access of the user profile data, genetic data, and other sensitive data. For example, the user may pre-authorize the access of the data and may change the setting as wish. The privacy settings also may allow a user to specify (e.g., by opting out, by not opting in) whether the computing server 130 may receive, collect, log, or store particular data associated with the user for any purpose. A user may restrict her data at various levels. For example, in one level, the data may not be accessed by the computing server 130 for purposes other than displaying the data in the user's own profile. On another level, the user may authorize anonymization of her data and participate in studies and researches conducted by the computing server 130 such as a large scale genetic study. In yet another level, the user may turn some portions of her genealogical data public to allow the user to be discovered by other users (e.g., potential relatives) and be connected in one or more family trees. Access or sharing of any information or data in the computing server 130 may also be subject to one or more similar privacy policies.

The sample pre-processing engine 215 receives and pre-processes data received from various sources to change the data into a format used by the computing server 130. For genealogical data, the sample pre-processing engine 215 may receive data from an individual via the user interface 115 of the client device 110. To collect the user data (e.g., genealogical and survey data), the computing server 130 may cause an interactive user interface on the client device 110 to display interface elements in which users can provide genealogical data and survey data. Additional data may be obtained from scans of public records. The data may be manually provided or automatically extracted via, for example, optical character recognition (OCR) performed on census records, town or government records, or any other item of printed or online material. Some records may be obtained by digitalizing written records such as older census records, birth certificates, death certificates, etc.

The sample pre-processing engine 215 may also receive raw data from genetic data extraction service server 125. The genetic data extraction service server 125 may perform laboratory analysis of biological samples of users and generate sequencing results in the form of digital data. The sample pre-processing engine 215 may receive the raw genetic datasets from the genetic data extraction service server 125. The human genome mutation rate is estimated to be 1.1*10^-8 per site per generation. This leads to a variant approximately every 300 base pairs. Most of the mutations that are passed down to descendants are related to single-nucleotide polymorphism (SNP). SNP is a substitution of a single nucleotide that occurs at a specific position in the genome. The sample pre-processing engine 215 may convert the raw base pair sequence into a sequence of genotypes of target SNP sites. Alternatively, the pre-processing of this conversion may be performed by the genetic data extraction service server 125. The sample pre-processing engine 215 identifies autosomal SNPs in an individual's genetic dataset. In one embodiment, the SNPs may be autosomal SNPs. In one embodiment, 700,000 SNPs may be identified in an individual's data and may be stored in genetic data store 205. Alternatively, in one embodiment, a genetic dataset may include at least 10,000 SNP sites. In another embodiment, a genetic dataset may include at least 100,000 SNP sites. In yet another embodiment, a genetic dataset may include at least 300,000 SNP sites. In yet another embodiment, a genetic dataset may include at least 1,000,000 SNP sites. The sample pre-processing engine 215 may also convert the nucleotides into bits. The identified SNPs, in bits or in other suitable formats, may be provided to the phasing engine 220 which phases the individual's diploid genotypes to generate a pair of haplotypes for each user.

The phasing engine 220 phases diploid genetic dataset into a pair of haploid genetic datasets and may perform imputation of SNP values at certain sites whose alleles are missing. An individual's haplotype may refer to a collection of alleles (e.g., a sequence of alleles) that are inherited from a parent.

Phasing may include a process of determining the assignment of alleles (particularly heterozygous alleles) to chromosomes. Owing to sequencing conditions and other constraints, a sequencing result often includes data regarding a pair of alleles at a given SNP locus of a pair of chromosomes but may not be able to distinguish which allele belongs to which specific chromosome. The phasing engine 220 uses a genotype phasing algorithm to assign one allele to a first chromosome and another allele to another chromosome. The genotype phasing algorithm may be developed based on an assumption of linkage disequilibrium (LD), which states that haplotype in the form of a sequence of alleles tends to cluster together. The phasing engine 220 is configured to generate phased sequences that are also commonly observed in many other samples. Put differently, haplotype sequences of different individuals tend to cluster together. A haplotype-cluster model may be generated to determine the probability distribution of a haplotype that includes a sequence of alleles. The haplotype-cluster model may be trained based on labeled data that includes known phased haplotypes from a trio (parents and a child). A trio is used as a training sample because the correct phasing of the child is almost certain by comparing the child's genotypes to the parent's genetic datasets. The haplotype-cluster model may be generated iteratively along with the phasing process with a large number of unphased genotype datasets. The haplotype-cluster model may also be used to impute one or more missing data.

By way of example, the phasing engine 220 may use a directed acyclic graph model such as a hidden Markov model (HMM) to perform phasing of a target genotype dataset. The directed acyclic graph may include multiple levels, each level having multiple nodes representing different possibilities of haplotype clusters. An emission probability of a node, which may represent the probability of having a particular haplotype cluster given an observation of the genotypes may be determined based on the probability distribution of the haplotype-cluster model. A transition probability from one node to another may be initially assigned to a non-zero value and be adjusted as the directed acyclic graph model and the haplotype-cluster model are trained. Various paths are possible in traversing different levels of the directed acyclic graph model. The phasing engine 220 determines a statistically likely path, such as the most probable path or a probable path that is at least more likely than 95% of other possible paths, based on the transition probabilities and the emission probabilities. A suitable dynamic programming algorithm such as the Viterbi algorithm may be used to determine the path. The determined path may represent the phasing result. U.S. Patent Application No. 15/591,099, entitled "Haplotype Phasing Models," filed on October 19, 2015, describes one possible embodiment of haplotype phasing.

The IBD estimation engine 225 estimates the amount of shared genetic segments between a pair of individuals based on phased genotype data (e.g., haplotype datasets) that are stored in the genetic data store 205. IBD segments may be segments identified in a pair of individuals that are putatively determined to be inherited from a common ancestor. The IBD estimation engine 225 retrieves a pair of haplotype datasets for each individual. The IBD estimation engine 225 may divide each haplotype dataset sequence into a plurality of windows. Each window may include a fixed number of SNP sites (e.g., about 100 SNP sites). The IBD estimation engine 225 identifies one or more seed windows in which the alleles at all SNP sites in at least one of the phased haplotypes between two individuals are identical. The IBD estimation engine 225 may expand the match from the seed windows to nearby windows until the matched windows reach the end of a chromosome or until a homozygous mismatch is found, which indicates the mismatch is not attributable to potential errors in phasing or in imputation. The IBD estimation engine 225 determines the total length of matched segments, which may also be referred to as IBD segments. The length may be measured in the genetic distance in the unit of centimorgans (cM). A unit of centimorgan may be a genetic length. For example, two genomic positions that are one cM apart may have a 1% chance during each meiosis of experiencing a recombination event between the two positions. The computing server 130 may save data regarding individual pairs who share a length of IBD segments exceeding a predetermined threshold (e.g., 6 cM), in a suitable data store such as in the genealogical data store 200. U.S. Patent Application No. 14/029,765, entitled "Identifying Ancestral Relationships Using a Continuous stream of Input," filed on September 17, 2013, and U.S. Patent Application No. 15/519,104, entitled "Reducing Error in Predicted Genetic Relationships," filed on April 13, 2017, describe example embodiments of IBD estimation.

Typically, individuals who are closely related share a relatively large number of IBD segments, and the IBD segments tend to have longer lengths (individually or in aggregate across one or more chromosomes). In contrast, individuals who are more distantly related share relatively fewer IBD segments, and these segments tend to be shorter (individually or in aggregate across one or more chromosomes). For example, while close family members often share upwards of 71 cM of IBD (e.g., third cousins), more distantly related individuals may share less than 12 cM of IBD. The extent of relatedness in terms of IBD segments between two individuals may be referred to as IBD affinity. For example, the IBD affinity may be measured in terms of the length of IBD segments shared between two individuals.

Community assignment engine 230 assigns individuals to one or more genetic communities based on the genetic data of the individuals. A genetic community may correspond to an ethnic origin or a group of people descended from a common ancestor. The granularity of genetic community classification may vary depending on embodiments and methods used in assigning communities. For example, in one embodiment, the communities may be African, Asian, European, etc. In another embodiment, the European community may be divided into Irish, German, Swedes, etc. In yet another embodiment, the Irish may be further divided into Irish in Ireland, Irish immigrated to America in 1800, Irish immigrated to America in 1900, etc. The community classification may also depend on whether a population is admixed or unadmixed. For an admixed population, the classification may further be divided based on different ethnic origins in a geographical region.

Community assignment engine 230 may assign individuals to one or more genetic communities based on their genetic datasets using machine learning models trained by unsupervised learning or supervised learning. In an unsupervised approach, the community assignment engine 230 may generate data representing a partially connected undirected graph. In this approach, the community assignment engine 230 represents individuals as nodes. Some nodes are connected by edges whose weights are based on IBD affinity between two individuals represented by the nodes. For example, if the total length of two individuals' shared IBD segments does not exceed a predetermined threshold, the nodes are not connected. The edges connecting two nodes are associated with weights that are measured based on the IBD affinities. The undirected graph may be referred to as an IBD network. The community assignment engine 230 uses clustering techniques such as modularity measurement (e.g., the Louvain method) to classify nodes into different clusters in the IBD network. Each cluster may represent a community. The community assignment engine 230 may also determine sub-clusters, which represent sub-communities. The computing server 130 saves the data representing the IBD network and clusters in the IBD network data store 235. U.S. Patent Application No. 15/168,011, entitled "Discovering Population Structure from Patterns of Identity-By-Descent," filed on May 28, 2016, describes one possible embodiment of community detection and assignment.

The community assignment engine 230 may also assign communities using supervised techniques. For example, genetic datasets of known genetic communities (e.g., individuals with confirmed ethnic origins) may be used as training sets that have labels of the genetic communities. Supervised machine learning classifiers, such as logistic regressions, support vector machines, random forest classifiers, and neural networks may be trained using the training set with labels. A trained classifier may distinguish binary or multiple classes. For example, a binary classifier may be trained for each community of interest to determine whether a target individual's genetic dataset belongs or does not belong to the community of interest. A multi-class classifier such as a neural network may also be trained to determine whether the target individual's genetic dataset most likely belongs to one of several possible genetic communities.

Reference panel sample store 240 stores reference panel samples for different genetic communities. A reference panel sample is a genetic data of an individual whose genetic data is the most representative of a genetic community. The genetic data of individuals with the typical alleles of a genetic community may serve as reference panel samples. For example, some alleles of genes may be over-represented (e.g., being highly common) in a genetic community. Some genetic datasets include alleles that are commonly present among members of the community. Reference panel samples may be used to train various machine learning models in classifying whether a target genetic dataset belongs to a community, in determining the ethnic composition of an individual, and in determining the accuracy in any genetic data analysis, such as by computing a posterior probability of a classification result from a classifier.

A reference panel sample may be identified in different ways. In one embodiment, an unsupervised approach in community detection may apply the clustering algorithm recursively for each identified cluster until the sub-clusters contain a number of nodes that is smaller than a threshold (e.g., contains fewer than 1000 nodes). For example, the community assignment engine 230 may construct a full IBD network that includes a set of individuals represented by nodes and generate communities using clustering techniques. The community assignment engine 230 may randomly sample a subset of nodes to generate a sampled IBD network. The community assignment engine 230 may recursively apply clustering techniques to generate communities in the sampled IBD network. The sampling and clustering may be repeated for different randomly generated sampled IBD networks for various runs. Nodes that are consistently assigned to the same genetic community when sampled in various runs may be classified as a reference panel sample. The community assignment engine 230 may measure the consistency in terms of a predetermined threshold. For example, if a node is classified to the same community 95% (or another suitable threshold) of times whenever the node is sampled, the genetic dataset corresponding to the individual represented by the node may be regarded as a reference panel sample. Additionally, or alternatively, the community assignment engine 230 may select N most consistently assigned nodes as a reference panel for the community.

Other ways to generate reference panel samples are also possible. For example, the computing server 130 may collect a set of samples and gradually filter and refine the samples until high-quality reference panel samples are selected. For example, a candidate reference panel sample may be selected from an individual whose recent ancestors are born at a certain birthplace. The computing server 130 may also draw sequence data from the Human Genome Diversity Project (HGDP). Various candidates may be manually screened based on their family trees, relatives' birth location, other quality control. Principal component analysis may be used to creates clusters of genetic data of the candidates. Each cluster may represent an ethnicity. The predictions of ethnicity of those candidates may be compared to the ethnicity information provided by the candidates to perform further screening.

The ethnicity estimation engine 245 estimates the ethnicity composition of a genetic dataset of a target individual. The genetic datasets used by the ethnicity estimation engine 245 may be genotype datasets or haplotype datasets. For example, the ethnicity estimation engine 245 estimates the ancestral origins (e.g., ethnicity) based on the individual's genotypes or haplotypes at the SNP sites. To take a simple example of three ancestral populations corresponding to African, European and Native American, an admixed user may have nonzero estimated ethnicity proportions for all three ancestral populations, with an estimate such as [0.05, 0.65, 0.30], indicating that the user's genome is 5% attributable to African ancestry, 65% attributable to European ancestry and 30% attributable to Native American ancestry. The ethnicity estimation engine 245 generates the ethnic composition estimate and stores the estimated ethnicities in a data store of computing server 130 with a pointer in association with a particular user.

In one embodiment, the ethnicity estimation engine 245 divides a target genetic dataset into a plurality of windows (e.g., about 1000 windows). Each window includes a small number of SNPs (e.g., 300 SNPs). The ethnicity estimation engine 245 may use a directed acyclic graph model to determine the ethnic composition of the target genetic dataset. The directed acyclic graph may represent a trellis of an inter-window hidden Markov model (HMM). The graph includes a sequence of a plurality of node groups. Each node group, representing a window, includes a plurality of nodes. The nodes representing different possibilities of labels of genetic communities (e.g., ethnicities) for the window. A node may be labeled with one or more ethnic labels. For example, a level includes a first node with a first label representing the likelihood that the window of SNP sites belongs to a first ethnicity and a second node with a second label representing the likelihood that the window of SNPs belongs to a second ethnicity. Each level includes multiple nodes so that there are many possible paths to traverses the directed acyclic graph.

The nodes and edges in the directed acyclic graph may be associated with different emission probabilities and transition probabilities. An emission probability associated with a node represents the likelihood that the window belongs to the ethnicity labeling the node given the observation of SNPs in the window. The ethnicity estimation engine 245 determines the emission probabilities by comparing SNPs in the window corresponding to the target genetic dataset to corresponding SNPs in the windows in various reference panel samples of different genetic communities stored in the reference panel sample store 240. The transition probability between two nodes represents the likelihood of transition from one node to another across two levels. The ethnicity estimation engine 245 determines a statistically likely path, such as the most probable path or a probable path that is at least more likely than 95% of other possible paths, based on the transition probabilities and the emission probabilities. A suitable dynamic programming algorithm such as the Viterbi algorithm or the forward-backward algorithm may be used to determine the path. After the path is determined, the ethnicity estimation engine 245 determines the ethnic composition of the target genetic dataset by determining the label compositions of the nodes that are included in the determined path. U.S. Patent Application No. 15/209,458, entitled "Local Genetic Ethnicity Determination System," filed on July 13, 2016, describes an example embodiment of ethnicity estimation.

The front-end interface 250 may display various results determined by the computing server 130. The results and data may include the IBD affinity between a user and another individual, the community assignment of the user, the ethnicity estimation of the user, phenotype prediction and evaluation, genealogical data search, family tree and pedigree, relative profile and other information. The front-end interface 250 may be a graphical user interface (GUI) that displays various information and graphical elements. The front-end interface 250 may take different forms. In one case, the front-end interface 250 may be a software application that can be displayed at an electronic device such as a computer or a smartphone. The software application may be developed by the entity controlling the computing server 130 and be downloaded and installed at the client device 110. In another case, the front-end interface 250 may take the form of a webpage interface of the computing server 130 that allows users to access their family tree and genetic analysis results through web browsers. In yet another case, the front-end interface 250 may provide an application program interface (API).

### FIRST EXAMPLE PROCESS To ANALYZE A TRAIT FROM PROFILE METADATA

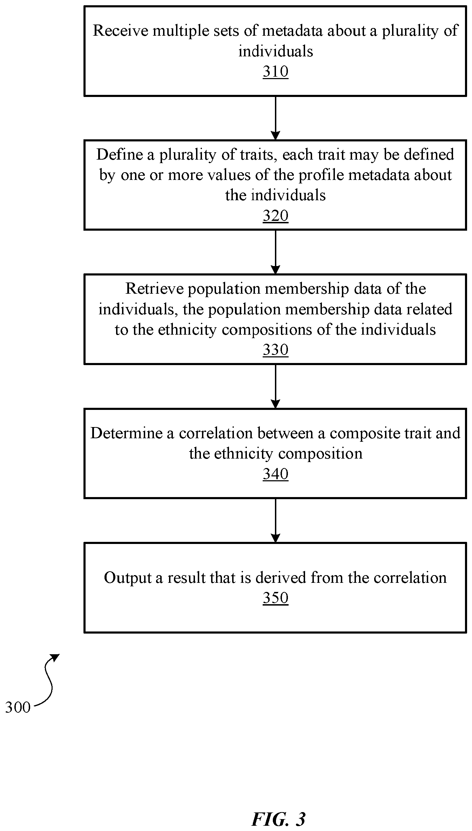

FIG. 3 is a flowchart illustrating an example process 300 to analyze a trait from profile metadata of an individual, in accordance with an embodiment. The computing server 130 may determine whether a particular genetic community (e.g., members with certain ethnicity compositions) may be correlated to a trait based on the process 300.

A target trait corresponds to a phenotype. For example, an example trait may be whether an individual has or does not have a certain appearance trait (e.g., hair curl, blue eye). Other suitable traits could include, but not limited to, wellness traits (e.g. male-pattern baldness), traits collected from historical records, traits collected from electronic health or electronic medical records, vitamin traits, sensitivity traits (e.g. aversion to cilantro), and sensory traits (e.g. ability to sense an asparagus metabolite). A trait used in the process 300 may also be a composite trait. A composite trait may be a particular combination of a group of traits. The group of traits may be identified from one or more sources of profile metadata about individuals. The sources may be survey responses, historical data, such as data of users' ancestors, and other suitable sources, including, but not limited to, medical records such as electronic health records, electronic medical records family health histories, census records, military service records, immigration records, birth, death or marriage certificates, photographs of individuals and their relatives and ancestors, and self-administered questionnaires from online surveys.

For example, in one embodiment, a composite trait may be defined by a combination of survey responses to multiple survey questions. In one embodiment, a composite trait may be defined by a series of questions that ask an individual regarding the type of trait of the individual and of the individual's relatives (e.g., parents and grandparents). For example, the series of questions may ask whether both the individual and one of individual's grandparents (or parents) speak the same language. If the responses for both questions regarding the individual and the individual's relatives are the same, a composite trait of language persistence in generations may be defined. Likewise, another composite trait of language discrepancy in generations may also be defined. Other types of composite traits related to language may also be possible. For instance, a first composite trait may be that both the individual and all of individual's grandparents speak the same language (e.g., English, Portuguese, etc.) A second composite trait may be that the individual speaks a first language and a maternal grandparent speaks a second language. The use of language to define a composite trait is discussed to illustrate various examples. A composite trait does not need to be related to language.

A composite trait may also be defined based on survey responses and at least another data value that is not derived from any survey responses. For example, historical data of an individual's ancestor may be obtained from a source other than surveys. In one case, a birth record of an ancestor may be obtained from census data. In another case, the health history record of an ancestor may be obtained from medical history. In yet another case, an appearance trait of an ancestor may be identified by a photo of the ancestor stored in the computing server 130. The computing server 130 may combine survey responses and data value that is not derived from survey responses to define a composite trait. For example, a composite trait may be that both the individual and one of individual's grandparents have blue eyes. The information regarding the individual's eye color may be obtained from the survey response while the information regarding the grandparent's eye color may be obtained from a photo.

A composite trait may represent phenotypes of individuals that are not easily representable by single data value. For example, a composite trait may represent that an individual has birthmarks, but the composite trait may also define the number of birthmarks, the colors of the birthmarks, and the locations of the birthmarks.

The metadata values (e.g., responses from different survey questions) that define a composite trait may have or may not have any apparent logical relationship. In one case, the data values are related. For example, a composite trait may be defined by multiple questions related to the languages spoken by the individual and the languages spoken by the individual's grandparents. In contrast, in another case, the data values that define a composite trait may be unrelated. For example, a composite trait may be defined by a data value related to a birthmark of an individual and another data value related to a language spoken by the individual.

The profile metadata about an individual may be retrieved from any suitable sources. The use of metadata retrieved from survey responses is merely one of the examples used for illustration. In one embodiment, none of the sources that define a composite trait may need to be related to or derived from survey responses. Also, the trait used in process 300 may not be a composite trait. For illustration, the example process 300 below is discussed with the use of survey responses to define a composite trait, but other sources of metadata about individuals may also be used to define a trait, composite or not.

In one embodiment, the computing server 130 may receive 310 multiple sets of metadata about a plurality of individuals. For example, each set of metadata may include or correspond to a set of survey responses obtained from an individual. Each set of survey responses may include responses to multiple survey questions. A set of metadata may also include other profile data obtained from other sources including, but not limited to, historical data, such as data of users' ancestors, medical records such as electronic health records, electronic medical records family health histories, census records, military service records, immigration records, birth, death or marriage certificates, photographs of individuals and their relatives and ancestors, and self-administered questionnaires from online surveys.

The computing server 130 may define 320 a plurality of traits. Each trait may be defined by one or more values of the profile metadata about the individuals. For example, a composite trait may be defined by a combination of survey responses to multiple survey questions. Defining a composite trait may depend completely on the values of profile metadata or only depend partially on the values. In one case, the composite trait may be defined completely by the combination of values. For example, if a composite trait is the native language of an individual and the native language of the individual's mother, the responses English-English for a first individual and the responses Portuguese-Portuguese for a second individual may be defined as two different composite traits. In contrast, in another case, the composite trait may be defined only partially based on the combination of values. The computing server 130 may classify certain combinations that share similar patterns as the same composite trait. For example, in the native language composite trait above, the computing server 130 may classify that any combinations that have the same value for both responses related to the individual and the individual's mother may be defined as the same trait. Hence, the first individual and the second individual may be classified as having the same composite trait under this definition. Other suitable ways to define a composite trait based on various combinations of profile metadata are also possible.

In defining different composite traits, the computing server 130 may construct a decision tree or a decision table to define the combinations as different composite traits. For example, a group of composite traits may be defined from three survey questions, which respectively have *m, n,* and *k* possible responses. In total, there can be *m* x *n* x *k* combinations of responses. The computing server 130 may construct, for example, a decision tree to classify the possible combinations into different branches and leaf nodes. In one case, each leaf node (e.g., a unique combination) may be defined as a separate composite trait. In another case, several leaf nodes included in a branch may be defined as a composite trait. In yet another case, other suitable ways to define composite traits are also possible.

The computing server 130 may retrieve 330 population membership data of the individuals. The population membership data may be data related to the genetic communities to which the individual belongs. Genetic communities may be defined based on the individuals' genetic data, such as the values of SNPs of the individuals. The population membership data may define different ethnicities, but may also be broader or narrower than ethnicities. For example, genetic communities may be divided on a continental level, a country level, a regional level, or any other suitable levels of granularity. In one embodiment, the population membership data may take the form of ethnicity composition. For example, the ethnicity estimation engine 245 may use a hidden Marko model (HMM) that divides the values of SNPs of an individual into a plurality of windows. Each window may include one or more ethnicity labels. The ethnicity estimation engine 245 may determine a statistically likely path that traverses the HMM and determined the ethnicity composition of the individual based on the compositions of ethnicity labels among different windows along the path. The ethnicity composition data of the individual may be saved as the population membership data in the profile of the individual. Alternatively, or additionally, the computing server 130 may use community assignment engine 230 to generate population membership data of the individuals. For example, the individuals may be classified into one or more genetic communities based on IBD connections among the individuals.

The computing server 130 may determine 340 a correlation between a composite trait and the ethnicity composition. For example, individuals may be classified by different composite traits. A subset of individuals may share the same composite trait while other individuals are not associated with the composite traits. Various statistics tool may be used to determine a potential correlation between the composite trait and the ethnicity composition of the individuals. Hypothesis testing and p-value determination may be used to examine any potential correlation and how strong the correlation is. Other statistical tools such as regressions may also be used. In one case, the population membership data may include classification data indicating that the individuals may belong to one or more genetic communities. An enrichment analysis may be performed to determine whether a particular composite trait is enriched within a certain genetic community. Enrichment analysis will be described in detail below. Other suitable methods to determine the relationship between a composite trait and a genetic community may also be used.

The computing server 130 may output 350 a result that is derived from the correlation. The result may be saved to a database of the computing server 130. For example, the computing server 130 may associate a composite trait, or traits included in the composite trait, with a particular genetic community such as an ethnicity. The result may also be transmitted for display at a graphical user interface of a client device 110. For example, a user of the computing server 130 may be classified into a particular genetic community. The computing server 130 may provide information regarding the increased likelihood of a particular trait associated with members of the genetic community.

### EXAMPLE STUDIES OF COMPOSITE TRAITS

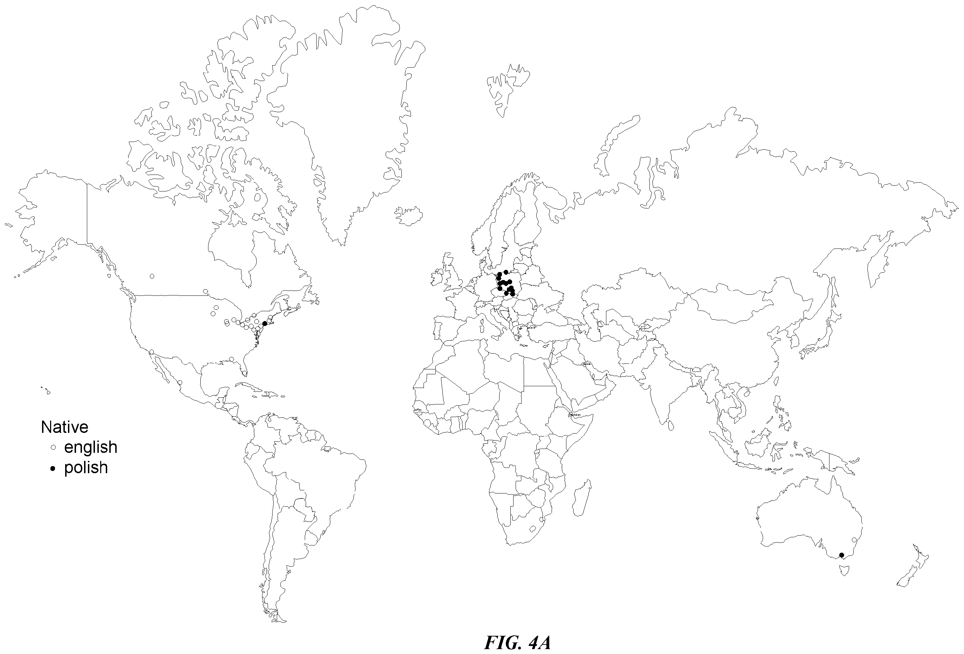

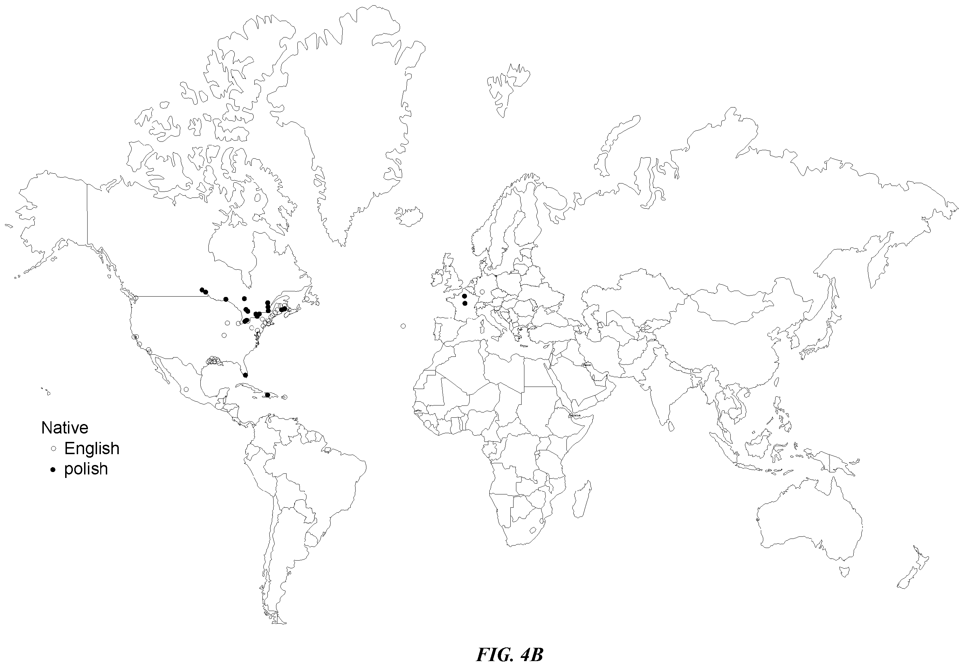

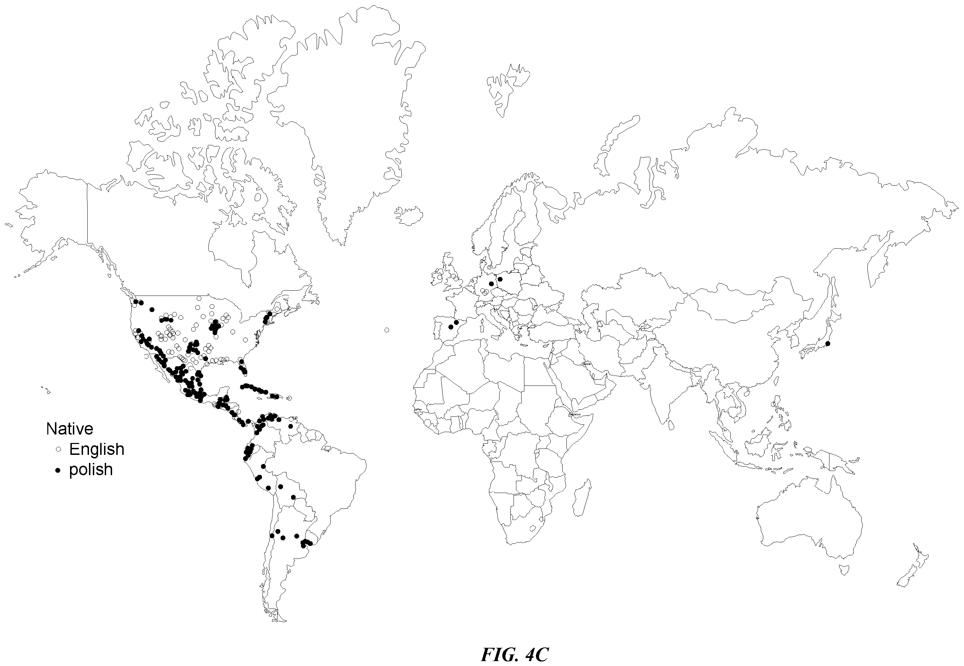

FIGS. 4A, 4B, and 4C are example geographical maps showing locations of birth of individuals whose grandparents speak a particular native language. The indicators on the maps are generated in accordance with an embodiment using at least some of the steps in process 300. The indicators are stratified into two sub-composite traits, depending on whether the individual speaks the same native language as the grandparents or not. FIG. 4A shows birth locations for individuals having the composite trait that all of the grandparents speak Polish. The individuals are stratified into a first subset whose members do not have the same native language as their grandparents (e.g., English speaking individuals) and a second subset whose members have the same native language as their grandparents (e.g., Polish-speaking individuals). FIG. 4B is similar to FIG. 4A, except the individuals have the composite trait that all of their grandparents speak French. Likewise, FIG. 4C shows birth locations of the individuals who have the composite trait that all of their grandparents speak Spanish and who are stratified into two subsets.

The results illustrated in FIGS. 4A, 4B, and 4C illustrate that this type of composite trait related to languages of the individuals and languages of the individuals' ancestors may provide some proxy for family history and migration patterns, especially for certain language such as Polish. The studies may be performed by surveying consenting individuals to ask the individuals' native languages, maternal grandparents' native languages, and paternal grandparents' native languages. The studies show that language persistence may be a strong proxy for local geography and potential history of movement.

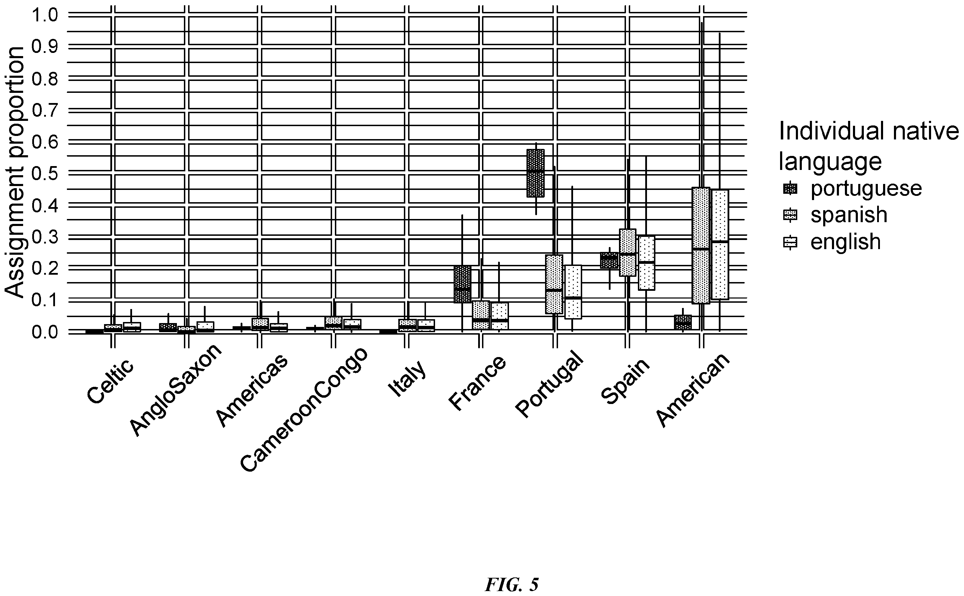

FIG. 5 is an example plot of the genetic ancestry composition of individuals with the composite trait that at least one of the grandparent of an individual speaks the same native language with the individual. The study was conducted using at least some of the steps described in process 300. Within a group of individuals with shared grandparent language, the study compares the estimates of genetic ancestry composition and study whether language tracks composition differences. The study shows that the genetic ancestry compositions of individuals with the composite trait of having one or more Portuguese speaking ancestors have significantly different from other individuals with other composite traits related to languages.

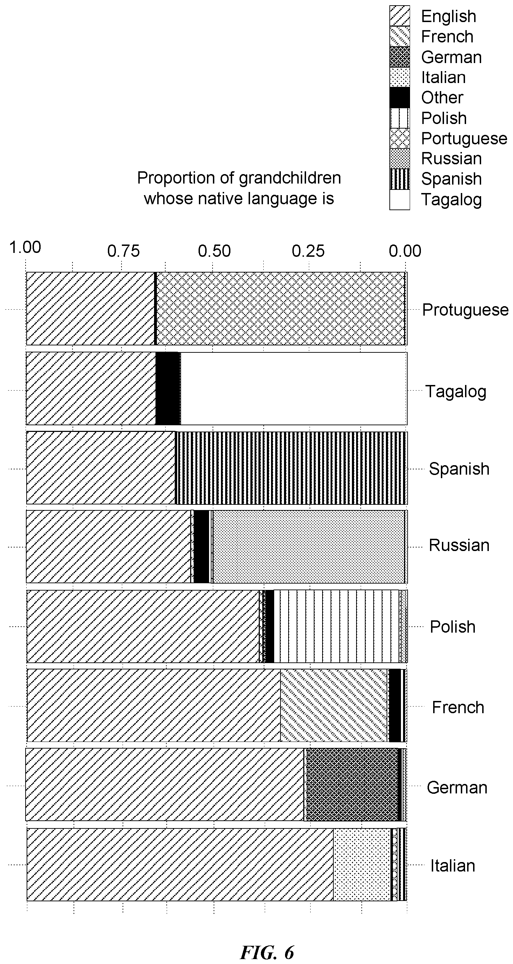

FIG. 6 is an example plot that illustrates the heritability of languages for individuals having the composite trait that two of their grandparents speak one language. The plot shows a proportion of the individuals (grandchildren) whose native language is one of a selection of nine or more languages. The study was conducted using at least some of the steps described in process 300. While individuals are likely to retain their grandparents' native languages as shown in the trend in FIG. 6, not all languages are retained equally. For instance, over 50% of individuals with Spanish-speaking grandparents speak Spanish. however, only 10% of individuals with Italian-speaking grandparents also speak Italian.

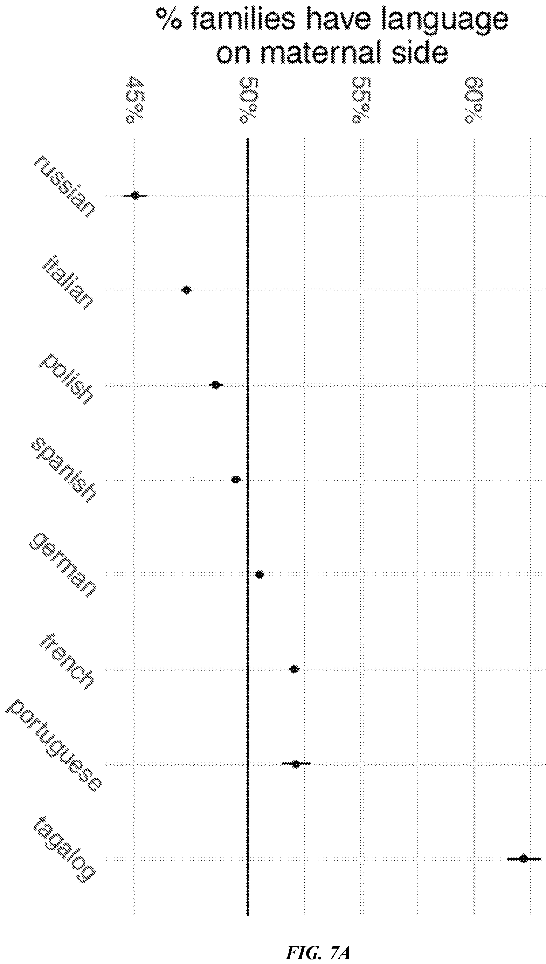

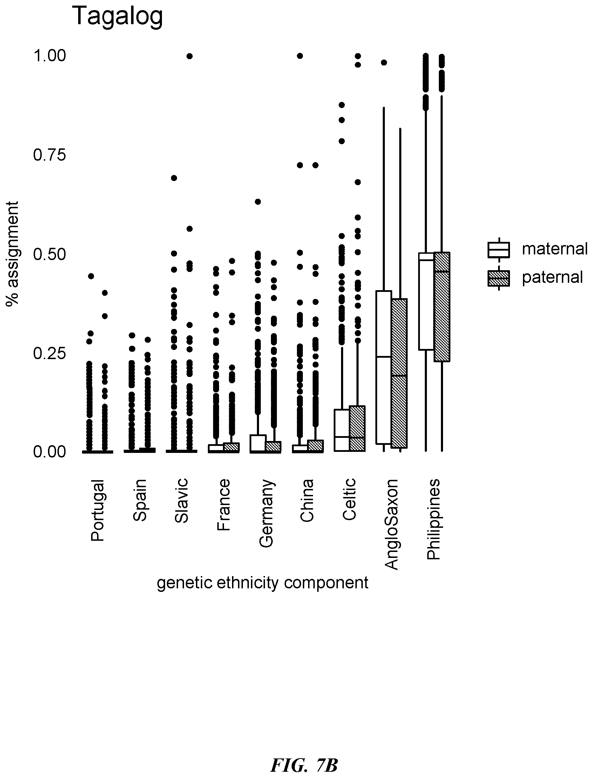

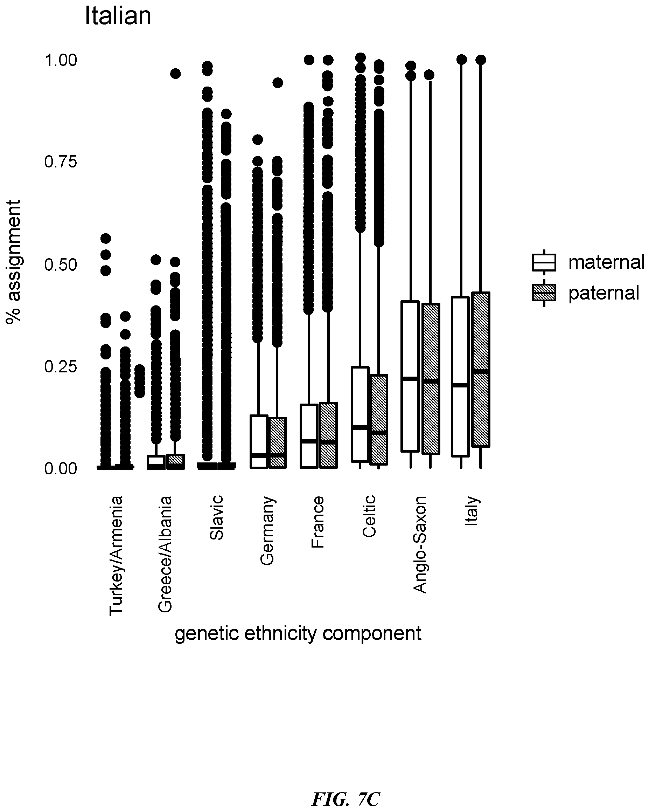

FIGS. 7A, 7B, 7C are example plots illustrating potential sex-bias in admixture individuals with composite traits that are defined based on maternal grandparent languages and paternal grandparent languages. The study was conducted using at least some of the steps described in process 300. The study identifies several examples of potential sex-biased admixture patterns. The signals may vary by language. In FIG. 7A, for a subset of languages on the x-axis, the y-axis shows the proportion of familial pairs where the maternal lineage and not the paternal lineage speaks that language. The bars denote standard error estimates. In FIGS. 7B and 7C, the study compares the genetic ancestry composition of individuals with disparate maternal and paternal side language history. In several examples, the study shows subtle though statistically significant differences in the distribution of assignments.

### SECOND EXAMPLE PROCESS To ANALYZE A TRAIT FROM PROFILE METADATA

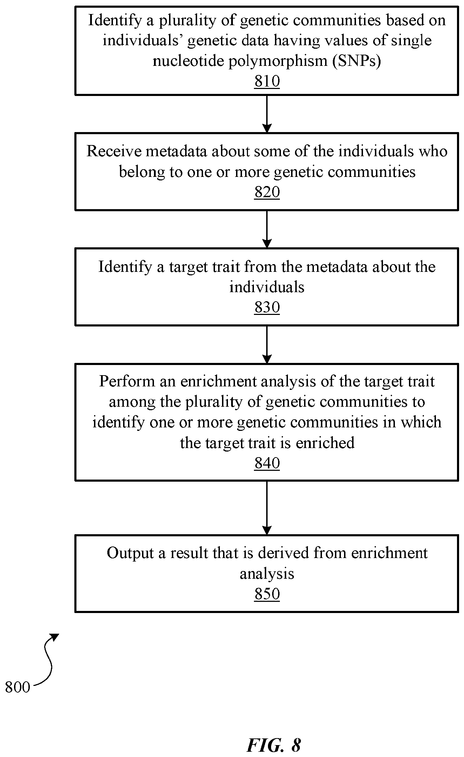

FIG. 8 is a flowchart illustrating an example process 800 to analyze a trait from profile metadata of an individual, in accordance with an embodiment. The process 800 may be used to study the prevalence of traits in genetic communities. In one embodiment, the process 800 may be used to identify a genetic community's enriched traits, which are traits that are particularly prevalent among members of the genetic community. The trait identified using process 800 may be a single trait (e.g., whether an individual has a phenotype or not) or a composite trait.

The computing server 130 may identify 810 a plurality of genetic communities. The genetic communities may be identified in any suitable way. For example, an individual associated with computing server 130 may have a profile stored in the computing server 130. The profile may include population membership data that has tags, attributes, or other suitable data values that identify the individual as members of one or more genetic communities. The membership of a genetic community of an individual may be determined based on the individual's genetic data and/or genealogical data such as pedigree or family tree data. In one embodiment, the computing server 130 may use community assignment engine 230 to separate multiple individuals into different genetic communities based on the individuals' values of SNPs. In another embodiment, the computing server 130 may use ethnicity estimation engine 245 to determine the ethnicity composition of an individual. Based on the ethnicity composition, the computing server 130 may assign the individual to one or more genetic communities.

The computing server 130 may receive 820 metadata about some of the individuals who belong to one or more genetic communities. For example, the computing server 130 may use population membership data to identify a group of individuals who belong to a genetic community. The computing server 130 may retrieve the survey responses from those individuals to identifies various traits possessed by those individuals. The survey questions may include one or more questions such as appearance trait questions, social-economical questions, cultural questions, preference questions, geographical questions, health-related questions, or family health history questions. The traits may also be identified by other metadata about the individuals that is not generated by survey responses. For example, the traits may be saved in the individuals' profiles as metadata that is generated from other sources including, but not limited to, historical data, such as data of users' ancestors, medical records such as electronic health records, electronic medical records family health histories, census records, military service records, immigration records, birth, death or marriage certificates, photographs of individuals and their relatives and ancestors, and self-administered questionnaires from online surveys. The computing server 130 may repeat the process to retrieve metadata about traits of members for other genetic communities.

The computing server 130 may identify 830 a target trait from the metadata about the individuals. The target trait may be derived from a single survey response, a number of survey responses, a combination of survey responses and other data, or data that is not derived from survey responses. The target trait is a phenotype (e.g., male baldness, eye color, presence or absence of a genetic disease) that may be determined by survey questions, by medical records, or by suitable sources. A target trait may also be a composite trait as discussed above. The target trait may be the possession of a certain trait, but may also be the lack of the trait.

The computing server 130 may perform 840 an enrichment analysis of the target trait among the plurality of genetic communities to identify one or more genetic communities in which the target trait is enriched. The target trait being enriched in a particular genetic community may indicate that the target trait statistically more likely to be present among the members of the genetic community compared to other genetic communities in which the target trait is not enriched. However, in some cases, the trait may be negatively defined (e.g., the lack of a particular phenotype) so that an enriched result may indicate that the genetic community has a reduced likelihood of possessing the trait. In one embodiment, the computing server 130 may also determine the correlation of the target trait in a genetic community. In one embodiment, the enrichment analysis may include Fisher's exact test to identify one or more genetic communities in which the target trait is enriched. In another embodiment, chi-square tests may be used to perform the enrichment analysis. Other suitable enrichment analyses may include the calculation of log odds ratios, measures of parametric and non-parametric correlations, such as Pearson's r, or Spearman's rho, to look at other kinds of enrichments or relationships. Alternatively, or additionally, statistics to assess correlation and association with more complex variables include Moran's I (operates on geographic relationships) or covariance calculations may also be used. In an enrichment analysis, the computing server 130 may determine an enrichment score for each genetic community. The enrichment score may represent a strength of association between the target trait and the genetic community. For example, the enrichment score may correspond to p-value of the observation of the target trait in a genetic community. In one embodiment, the enrichment score may take the form of the odds ratio of a particular genetic community having the target trait. The computing server 130 may compare the odds ratios among various genetic communities. Genetic communities with the odds ratios that exceed a certain threshold and that are within the top certain percentile may be determined as the target trait being enriched. Conversely, genetic communities with the odds ratios that are below another threshold or at certain bottom percentile may be determined as communities that have reduced likelihoods of possessing the trait. Other suitable statistical methods may be used to conduct the enrichment analysis.

The computing server 130 may output 850 a result that is derived from enrichment analysis. The result may be saved to a database of the computing server 130. For example, the computing server 130 may associate a trait with a particular genetic community. The result may also be transmitted for display at a graphical user interface of a client device 110. For example, a user of the computing server 130 may be classified into a particular genetic community. The computing server 130 may provide information regarding the increased likelihood of a particular trait associated with members of the genetic community. For example, in one case, the computing server 130 may provide information that is a certain ethnicity may be associated with an increased likelihood of male baldness.

In one embodiment, the computing server 130 may also use the enrichment result to predict whether an individual has a trait. For example, the computing server 130 may use the genetic data of the individual to determine that the individual should belong to one or more genetic communities. If the trait that the computing server 130 attempts to predict is enriched to one of the individual's genetic community, the computing server 130 may provide a prediction, such as in terms of probability or another similar value such as an odds ratio, of whether the individual possesses the trait. The computing server 130 may also refer to other data to verify or confirm the prediction or may rely on other data in addition to the enrichment result to generate the prediction. For example, the computing server 130 may rely on genealogical data and family tree to determine how common the trait is among the close relatives or other family members of the individual.

### EXAMPLE STUDIES OF ENRICHMENT ANALYSES

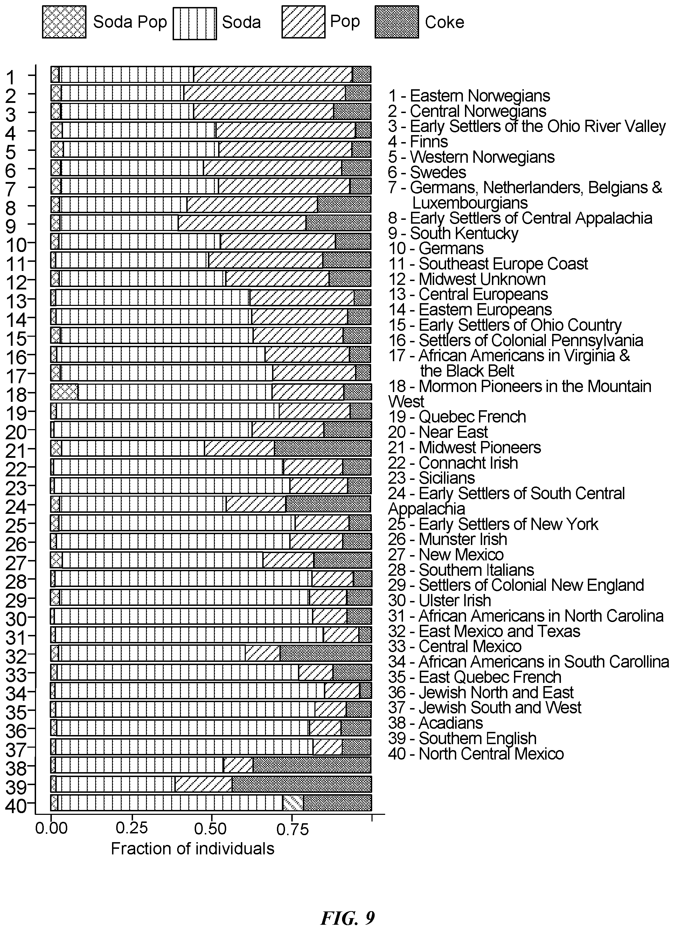

FIG. 9 is an example plot showing the preference of sweetened carbonated beverages for members of different genetic communities. The plot may be generated in accordance with an embodiment using at least some of the steps in the process 800. The preference data may be obtained from survey responses of individuals. The study in FIG. 9 shows that a certain preference of beverages may be enriched among members of the certain genetic community (e.g., Southern Italian) who may particularly enjoy soda compared to other genetic communities.

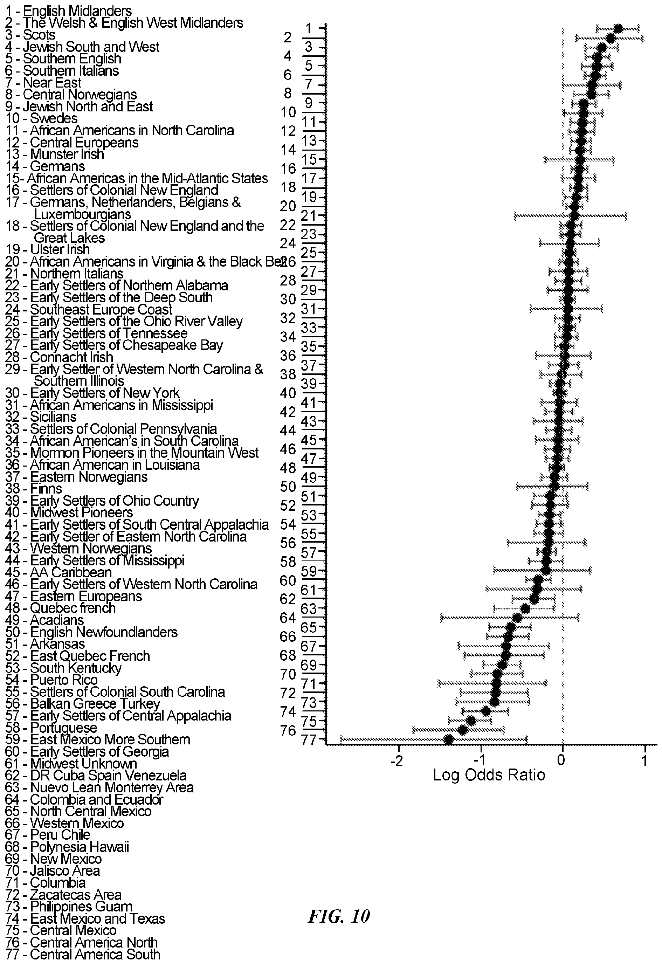

FIG. 10 is an example plot showing the log odds ratios of male-pattern baldness for different genetic communities. The plot may be generated in accordance with an embodiment using at least some of the steps in the process 800. The data regarding the phenotype of baldness may be obtained from survey responses of individuals or other suitable sources, such as photos of the individuals. The study in FIG. 10 shows that a certain phenotype may be enriched among members of certain genetic communities, while members of other genetic communities may have reduced the likelihoods of expressing the phenotype.

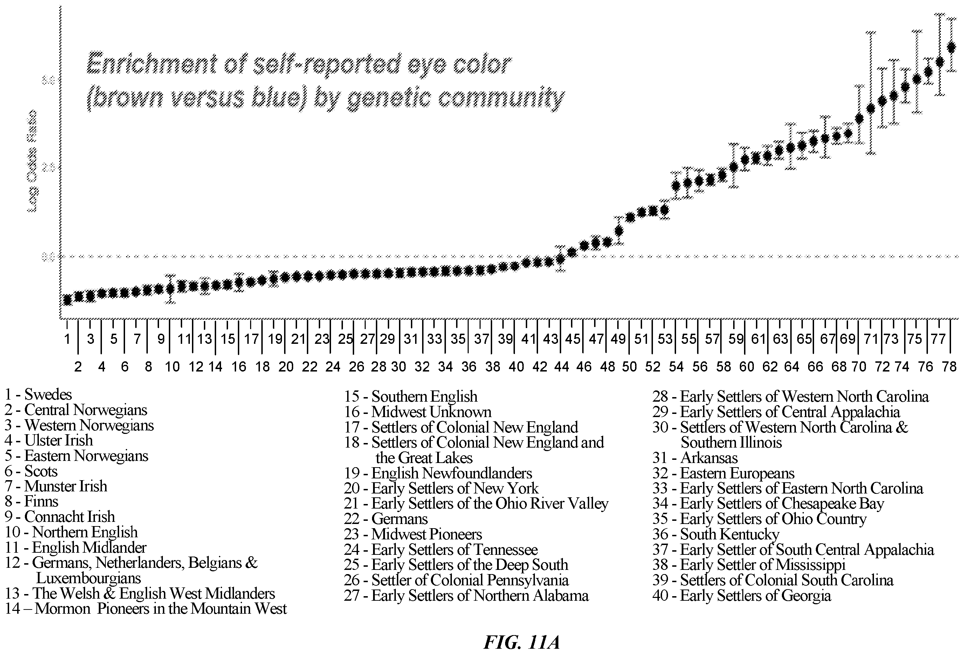

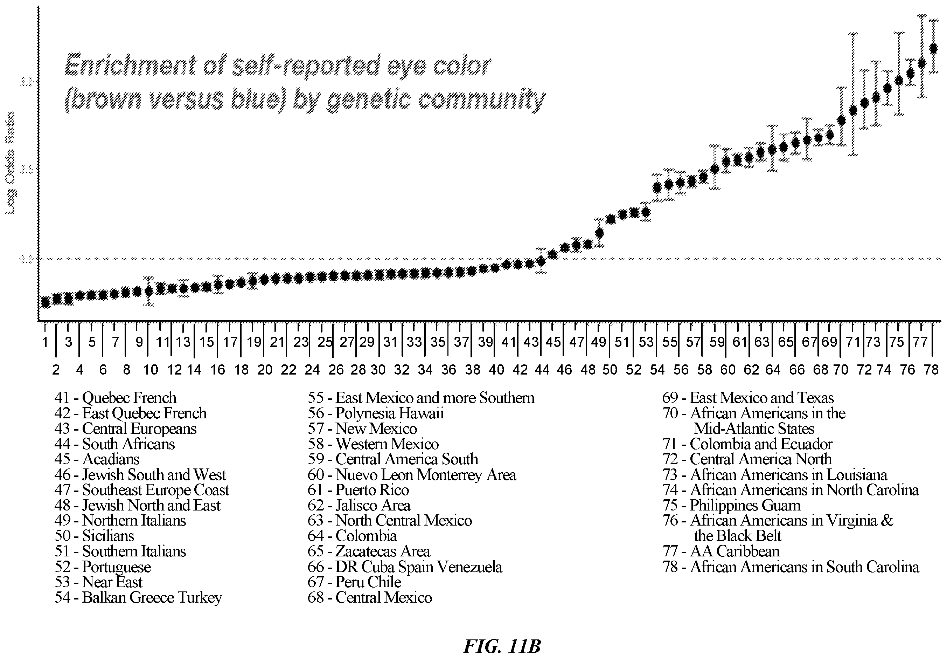

FIGS. 11A and 11B are an example plots showing the log odds ratios of brown eyes versus blue eyes for different genetic communities. FIG. 11A shows the first forty labels of genetic communities and FIG. 11B is the same plot showing the rest of the labels of genetic communities. The plot may be generated in accordance with an embodiment using at least some of the steps in the process 800. The data regarding the phenotype of eye color may be obtained from survey responses of individuals or other suitable sources, such as photos of the individuals. A negative log odds ratio may indicate a higher likelihood of having blue eyes while a positive odds ratio may indicate a higher likelihood of having brown eyes.

### EXAMPLE ASSOCIATION STUDIES

The computing server 130 may also perform association studies of a target trait using genetic data of individuals in one of the genetic communities to identify one or more genetic loci that may be associated with the target trait, in accordance with an embodiment. By way of example, the computing server 130 may determine that a target trait is enriched with a target genetic community. The computing server 130 may retrieve population membership data of individuals' profiles saved in the computing server 130. The population membership data may be determined by the ethnicity estimation engine 245, which may include a hidden Marko model to divide the values of SNPs of individuals into a plurality of windows to determine the ethnicity compositions of the individuals. For the individuals who have portions of the target ethnicity that exceed a threshold, the individuals may be labeled as being part of the genetic community of the target ethnicity. The computing server 130 may use the genetic data for those individuals who belong to the genetic community to perform an association study of a target trait. The association study may identify one or more genetic loci that are associated with the target trait. In one embodiment, the association may be a genome-wide association study (GWAS). Details of example association studies are further discussed in U.S. Patent Application No., 16/598,988, filed on October 10, 2019, entitled "Phenotype Trait Prediction with Threshold Polygenic Risk Score."

By way of example, in conducting an association study, the computing server 130 may obtain data of a plurality of training individuals that belong to the target genetic community. The training individuals include at least a first subset of positive training individuals, (e.g., training individuals reported to have the target trait in consideration) and a second subset of negative training individuals (e.g., training individuals reported to not have the phenotypic trait). Each training individual has a genetic dataset including values of SNPs over a plurality of genetic loci. The value on whether or not that an individual has a trait may be retrieved from the metadata about the individual.

The computing server 130 may conduct an association study for a trait with the plurality of training individuals. In conducting the association study, the computing server 130, at each genetic locus in the genetic datasets of the training individuals, may count a first percentage of positive training individuals with a genotype including the SNP and counts a second percentage of negative training individuals with a genotype including the SNP. The computing server 130 may calculate a predictive score (e.g., by conducting an enrichment analysis such as the Fisher's Exact Test) for each genetic locus according to the first percentage and the second percentage, e.g., an odds ratio of the first percentage to the second percentage. In some embodiments, the computing server 130 calculates a p-value score as the predictive score, wherein the p-value score indicates a likelihood of observing a result that is equal to or more extreme than the actual observed percentages. The computing server 130 may tabulate the predictive scores (e.g., p-value scores) for each genetic locus. The computing server 130 may, in turn, identify SNP(s) at one or more genetic loci that are most strongly associated with the target trait.

The result of an association study may be used to generate a prediction of a trait of a target individual (e.g., a new user). For example, the computing server 130 may receive a genetic dataset of the target individual. The genetic dataset may include values of SNPs of the target individual. The computing server 130 may predict whether the target individual possesses the target trait based on the values of the SNPs of the target individual in the one or more genetic loci that are identified in the association study. For example, if the target individual has the SNP values that are highly associated with the trait at the identified loci, the target individual may be predicted to have the trait. A predictive score, which may be a weighted or aggregated value of the presence or absence of certain SNP values at the identified loci, may be generated based on the genetic data of the target individual.

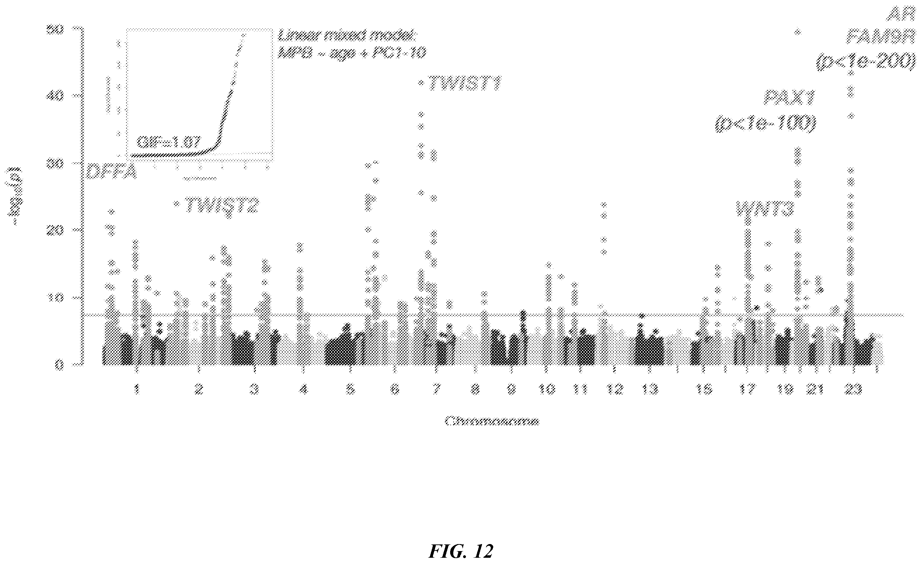

FIG. 12 is an example plot of a result of an association study of male-pattern baldness of the genetic community of a cohort of individuals of European ancestry. The association study is conducted in accordance with an embodiment. In one example study, 10,508 cases of positive training individuals and 35,003 of negative training individuals are used in this study. The result of the study shows that certain genetic loci, such as DFFA, TWIST2, TTWIST1, WNT3, PAX1, and AR-FAM9R, are more associated with the male baldness trait compared to other genetic loci.

### COMPUTING MACHINE ARCHITECTURE

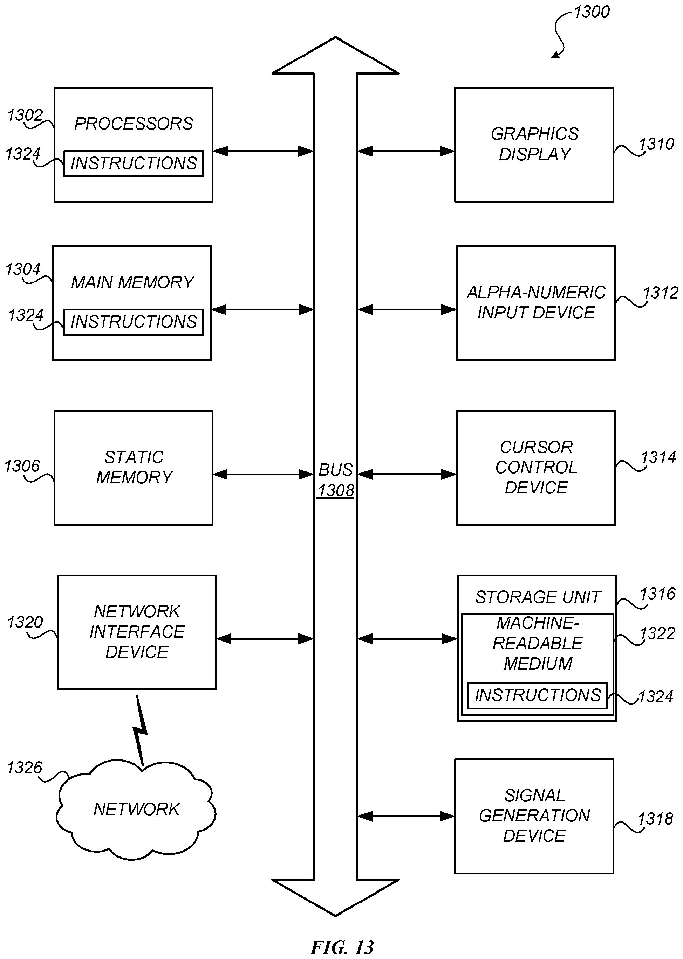

FIG. 13 is a block diagram illustrating components of an example computing machine that is capable of reading instructions from a computer-readable medium and execute them in a processor (or controller). A computer described herein may include a single computing machine shown in FIG. 13, a virtual machine, a distributed computing system that includes multiples nodes of computing machines shown in FIG. 13, or any other suitable arrangement of computing devices.

By way of example, FIG. 13 shows a diagrammatic representation of a computing machine in the example form of a computer system 1300 within which instructions 1324 (e.g., software, program code, or machine code), which may be stored in a computer-readable medium for causing the machine to perform any one or more of the processes discussed herein may be executed. In some embodiments, the computing machine operates as a standalone device or may be connected (e.g., networked) to other machines. In a networked deployment, the machine may operate in the capacity of a server machine or a client machine in a server-client network environment, or as a peer machine in a peer-to-peer (or distributed) network environment.

The structure of a computing machine described in FIG. 13 may correspond to any software, hardware, or combined components shown in FIGS. 1 and 2, including but not limited to, the client device 110, the computing server 130, and various engines, interfaces, terminals, and machines shown in FIG. 2. While FIG. 13 shows various hardware and software elements, each of the components described in FIGS. 1 and 2 may include additional or fewer elements.

By way of example, a computing machine may be a personal computer (PC), a tablet PC, a set-top box (STB), a personal digital assistant (PDA), a cellular telephone, a smartphone, a web appliance, a network router, an internet of things (IoT) device, a switch or bridge, or any machine capable of executing instructions 1324 that specify actions to be taken by that machine. Further, while only a single machine is illustrated, the term "machine" and "computer" may also be taken to include any collection of machines that individually or jointly execute instructions 1324 to perform any one or more of the methodologies discussed herein.

The example computer system 1300 includes one or more processors 1302 such as a CPU (central processing unit), a GPU (graphics processing unit), a TPU (tensor processing unit), a DSP (digital signal processor), a system on a chip (SOC), a controller, a state equipment, an application-specific integrated circuit (ASIC), a field-programmable gate array (FPGA), or any combination of these. Parts of the computing system 1300 may also include a memory 1304 that store computer code including instructions 1324 that may cause the processors 1302 to perform certain actions when the instructions are executed, directly or indirectly by the processors 1302. Instructions can be any directions, commands, or orders that may be stored in different forms, such as equipment-readable instructions, programming instructions including source code, and other communication signals and orders. Instructions may be used in a general sense and are not limited to machine-readable codes.

One and more methods described herein improve the operation speed of the processors 1302 and reduces the space required for the memory 1304. For example, the machine learning methods described herein reduces the complexity of the computation of the processors 1302 by applying one or more novel techniques that simplify the steps in training, reaching convergence, and generating results of the processors 1302. The algorithms described herein also reduces the size of the models and datasets to reduce the storage space requirement for memory 1304.

The performance of certain of the operations may be distributed among the more than processors, not only residing within a single machine, but deployed across a number of machines. In some example embodiments, the one or more processors or processor-implemented modules may be located in a single geographic location (e.g., within a home environment, an office environment, or a server farm). In other example embodiments, the one or more processors or processor-implemented modules may be distributed across a number of geographic locations. Even though in the specification or the claims may refer some processes to be performed by a processor, this should be construed to include a joint operation of multiple distributed processors.

The computer system 1300 may include a main memory 1304, and a static memory 1306, which are configured to communicate with each other via a bus 1308. The computer system 1300 may further include a graphics display unit 1310 (*e.g.,* a plasma display panel (PDP), a liquid crystal display (LCD), a projector, or a cathode ray tube (CRT)). The graphics display unit 1310, controlled by the processors 1302, displays a graphical user interface (GUI) to display one or more results and data generated by the processes described herein. The computer system 1300 may also include alphanumeric input device 1312 (e.g., a keyboard), a cursor control device 1314 (e.g., a mouse, a trackball, a joystick, a motion sensor, or other pointing instrument), a storage unit 1316 (a hard drive, a solid state drive, a hybrid drive, a memory disk, etc.), a signal generation device 1318 (e.g., a speaker), and a network interface device 1320, which also are configured to communicate via the bus 1308.

The storage unit 1316 includes a computer-readable medium 1322 on which is stored instructions 1324 embodying any one or more of the methodologies or functions described herein. The instructions 1324 may also reside, completely or at least partially, within the main memory 1304 or within the processor 1302 (e.g., within a processor's cache memory) during execution thereof by the computer system 1300, the main memory 1304 and the processor 1302 also constituting computer-readable media. The instructions 1324 may be transmitted or received over a network 1326 via the network interface device 1320.

While computer-readable medium 1322 is shown in an example embodiment to be a single medium, the term "computer-readable medium" should be taken to include a single medium or multiple media (e.g., a centralized or distributed database, or associated caches and servers) able to store instructions (e.g., instructions 1324). The computer-readable medium may include any medium that is capable of storing instructions (e.g., instructions 1324) for execution by the processors (e.g., processors 1302) and that causes the processors to perform any one or more of the methodologies disclosed herein. The computer-readable medium may include, but not be limited to, data repositories in the form of solid-state memories, optical media, and magnetic media. The computer-readable medium does not include a transitory medium such as a propagating signal or a carrier wave.

### ADDITIONAL CONSIDERATIONS

The foregoing description of the embodiments has been presented for the purpose of illustration; it is not intended to be exhaustive or to limit the patent rights to the precise forms disclosed. Persons skilled in the relevant art can appreciate that many modifications and variations are possible in light of the above disclosure.

The subject-matter may include not only the combinations of features as set out in the disclosed embodiments but also any other combination of features from different embodiments. Various features mentioned in the different embodiments can be combined with explicit mentioning of such combination or arrangement in an example embodiment or without any explicit mentioning.

Some portions of this description describe the embodiments in terms of algorithms and symbolic representations of operations on information. These operations and algorithmic descriptions, while described functionally, computationally, or logically, are understood to be implemented by computer programs or equivalent electrical circuits, microcode, or the like. Furthermore, it has also proven convenient at times, to refer to these arrangements of operations as engines, without loss of generality. The described operations and their associated engines may be embodied in software, firmware, hardware, or any combinations thereof.

Any of the steps, operations, or processes described herein may be performed or implemented with one or more hardware or software engines, alone or in combination with other devices. In one embodiment, a software engine is implemented with a computer program product comprising a computer-readable medium containing computer program code, which can be executed by a computer processor for performing any or all of the steps, operations, or processes described. The term "steps" does not mandate or imply a particular order. For example, while this disclosure may describe a process that includes multiple steps sequentially with arrows present in a flowchart, the steps in the process do not need to be performed by the specific order claimed or described in the disclosure. Some steps may be performed before others even though the other steps are claimed or described first in this disclosure. Likewise, any use of (i), (ii), (iii), etc., or (a), (b), (c), etc. in the specification or in the claims, unless specified, is used to better enumerate items or steps and also does not mandate a particular order.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein. In claims, the use of a singular form of a noun may imply at least one element even though a plural form is not used.

Finally, the language used in the specification has been principally selected for readability and instructional purposes, and it may not have been selected to delineate or circumscribe the patent rights. It is therefore intended that the scope of the patent rights be limited not by this detailed description, but rather by any claims that issue on an application based hereon. Accordingly, the disclosure of the embodiments is intended to be illustrative, but not limiting, of the scope of the patent rights.

## Claims

1. A computer-implemented method, comprising:
identifying a plurality of genetic communities, each genetic community comprising individuals identified based on values of single nucleotide polymorphisms (SNPs) of the individuals;
receiving metadata about some of the individuals who belong to one or more of the plurality of genetic communities;
identifying a target trait derived from the metadata, wherein the target trait is a phenotype;
performing, for the target trait, an enrichment analysis among the plurality of genetic communities to identify one or more genetic communities in which the target trait is enriched, the target trait being enriched in a particular genetic community indicating that the target trait is statistically more likely to be present among the members of the particular genetic community compared to other genetic communities in which the target trait is not enriched;
outputting a result derived from the enrichment analysis;
performing an association study of the target trait using genetic data of the individuals in one of the genetic communities in which the target trait is enriched to identify one or more genetic loci that are associated with the target trait;
receiving a genetic dataset of a target individual, the genetic dataset including values of SNPs of the target individual; and
predicting whether the target individual possesses the target trait based on values of the SNPs of the target individual in the identified one or more genetic loci.

2. The computer-implemented method of claim 1, wherein the target trait is a composite trait that is defined at least partially by a combination of a plurality of survey responses.

3. The computer-implemented method of claim 2, wherein the composite trait is defined by a first response to a first survey question related to a type of trait of the plurality of individuals and a second response to a second survey question related to the same type of trait of parents or grandparents of the plurality of individuals.

4. The computer-implemented method of claim 3, wherein the composite trait is defined by the first response being the same as the second response.

5. The computer-implemented method of claim 2, wherein the composite trait is defined at least by a survey response and another data value that is not derived from any survey responses.

6. The computer-implemented method of claim 1, wherein the enrichment analysis includes a Fisher's exact test.

7. The computer-implemented method of claim 1, wherein the association study is a genome-wide association study (GWAS).

8. The computer-implemented method of claim 1, wherein the plurality of genetic communities are identified based on identity by descent (IBD) connections among the individuals.

9. The computer-implemented method of claim 1, wherein the plurality of genetic communities are identified based on a hidden Markov model which divides the values of SNPs of each individual into a plurality of windows, each window comprising one or more ethnicity labels.

10. The computer-implemented method of claim 1, wherein at least some of the metadata are derived from survey responses comprising responses to one or more survey questions, the one or more survey questions comprising one or more of the following: an appearance trait question, a social-economical question, a cultural question, a preference question, a geographical question, a health-related question, or a family health history question.

11. A non-transitory computer readable medium configured to store instructions, the instructions, when executed by one or more processors, cause the one or more processors to perform a process comprising steps recited in any of claims 1-10.

12. A system comprising:
one or more processors; and
a storage medium configured to store instructions, the instructions, when executed by the one or more processors, cause the one or more processors to perform a process comprising steps recited in any of claims 1-10.

## Patentansprüche

1. Computergestütztes Verfahren mit den Schritten:
Identifizieren einer Mehrzahl genetischer Gemeinschaften, wobei jede genetische Gemeinschaft Individuen umfasst, die auf der Grundlage von Werten von Einzelnukleotid-Polymorphismen (SNPs) der Individuen identifiziert werden;
Empfangen von Metadaten über einige der Individuen, die zu einer oder mehreren der Mehrzahl genetischer Gemeinschaften gehören;
Identifizieren eines aus den Metadaten abgeleiteten Zielmerkmals, wobei das Zielmerkmal ein Phänotyp ist;
Durchführen einer Anreicherungsanalyse für das Zielmerkmal unter den mehreren genetischen Gemeinschaften, um eine oder mehrere genetische Gemeinschaften zu identifizieren, in denen das Zielmerkmal angereichert ist, wobei eine Anreicherung des Zielmerkmals in einer bestimmten genetischen Gemeinschaft anzeigt, dass das Zielmerkmal statistisch gesehen mit größerer Wahrscheinlichkeit unter den Mitgliedern der bestimmten genetischen Gemeinschaft vorhanden ist als in anderen genetischen Gemeinschaften, in denen das Zielmerkmal nicht angereichert ist;
Ausgeben eines aus der Anreicherungsanalyse abgeleiteten Ergebnisses;
Durchführen einer Assoziationsstudie des Zielmerkmals unter Verwendung genetischer Daten der Individuen in einer der genetischen Gemeinschaften, in denen das Zielmerkmal angereichert ist, um einen oder mehrere genetische Loci zu identifizieren, die mit dem Zielmerkmal assoziiert sind;
Empfangen eines genetischen Datensatzes eines Zielindividuums, wobei der genetische Datensatz Werte von SNPs des Zielindividuums enthält; und
Vorhersagen, ob das Zielindividuum das Zielmerkmal besitzt, basierend auf Werten der SNPs des Zielindividuums an den identifizierten ein oder mehreren genetischen Loci.

2. Computergestütztes Verfahren nach Anspruch 1, bei dem das Zielmerkmal ein zusammengesetztes Merkmal ist, das zumindest teilweise durch eine Kombination einer Mehrzahl von Umfrageantworten definiert ist.

3. Computergestütztes Verfahren nach Anspruch 2, bei dem das zusammengesetzte Merkmal definiert ist durch eine erste Antwort auf eine erste Umfragefrage, die sich auf einen Merkmalstyp der Mehrzahl von Individuen bezieht, und eine zweite Antwort auf eine zweite Umfragefrage, die sich auf denselben Merkmalstyp von Eltern oder Großeltern der Mehrzahl von Individuen bezieht.

4. Computergestütztes Verfahren nach Anspruch 3, bei dem das zusammengesetzte Merkmal dadurch definiert ist, dass die erste Antwort mit der zweiten Antwort übereinstimmt.

5. Computergestütztes Verfahren nach Anspruch 2, bei dem das zusammengesetzte Merkmal mindestens durch eine Umfrageantwort und einen anderen, nicht aus Umfrageantworten abgeleiteten Datenwert definiert ist.

6. Computergestütztes Verfahren nach Anspruch 1 , bei dem die Anreicherungsanalyse einen exakten Test nach Fisher umfasst.

7. Computergestütztes Verfahren nach Anspruch 1, bei dem die Assoziationsstudie eine genomweite Assoziationsstudie (GWAS) ist.

8. Computergestütztes Verfahren nach Anspruch 1, bei dem die Mehrzahl genetischer Gemeinschaften auf der Grundlage von Verwandtschaftsbeziehungen (IBD-Verbindungen) zwischen den Individuen identifiziert wird.

9. Computergestütztes Verfahren nach Anspruch 1, bei dem die Mehrzahl genetischer Gemeinschaften auf der Grundlage eines versteckten Markov-Modells identifiziert wird, das die Werte von SNPs jedes Individuums in eine Mehrzahl von Fenstern unterteilt, wobei jedes Fenster eine oder mehrere Ethnizitätskennungen aufweist.

10. Computerimplementiertes Verfahren nach Anspruch 1, bei dem mindestens einige der Metadaten aus Umfrageantworten abgeleitet werden, die Antworten auf eine oder mehrere Fragen umfassen, wobei die eine oder mehreren Fragen eine oder mehrere der folgenden Fragen umfassen: eine Frage zu einem äußerlichen Merkmal, eine sozioökonomische Frage, eine kulturelle Frage, eine Frage zu Vorlieben, eine geografische Frage, eine Frage zu Gesundheit oder eine Frage zur Familiengesundheitsgeschichte.

11. Nichtflüchtiges computerlesbares Medium, das zum Speichern von Anweisungen konfiguriert ist, wobei die Anweisungen, wenn sie von einem oder mehreren Prozessoren ausgeführt werden, die ein oder mehreren Prozessoren veranlassen, einen Prozess auszuführen, der in einem der Ansprüche 1-10 genannte Schritte umfasst.

12. System mit:
einem oder mehreren Prozessoren; und
einem Speichermedium, das zum Speichern von Anweisungen konfiguriert ist, wobei die Anweisungen, wenn sie von den ein oder mehreren Prozessoren ausgeführt werden, bewirken, dass die ein oder mehreren Prozessoren einen Prozess ausführen, der in einem der Ansprüche 1-10 genannte Schritte umfasst.

## Revendications

1. Procédé mis en oeuvre par ordinateur, comprenant :
l'identification d'une pluralité de communautés génétiques, chaque communauté génétique comprenant des individus identifiés sur la base de valeurs de polymorphismes mononucléotidiques (SNP) des individus ;
la réception de métadonnées au sujet de certains des individus qui appartiennent à une ou plusieurs de la pluralité de communautés génétiques ;
l'identification d'un caractère cible déduit des métadonnées, dans lequel le caractère cible est un phénotype ;
la réalisation, pour le caractère cible, d'une analyse d'enrichissement parmi la pluralité de communautés génétiques pour identifier une ou plusieurs communautés génétiques dans lesquelles le caractère cible est enrichi, le caractère cible étant enrichi dans une communauté génétique particulière indiquant que le caractère cible présente une plus grande probabilité statistique d'être présent parmi les membres de la communauté génétique particulière par comparaison à d'autres communautés génétiques dans lesquelles le caractère cible n'est pas enrichi ;
la délivrance d'un résultat déduit de l'analyse d'enrichissement ;
la réalisation d'une étude d'association du caractère cible à l'aide de données génétiques des individus dans l'une des communautés génétiques dans lesquelles le caractère cible est enrichi pour identifier un ou plusieurs loci génétiques qui sont associés au caractère cible ;
la réception d'un jeu de données génétiques d'un individu cible, le jeu de données génétiques comportant des valeurs de SNP de l'individu cible ; et
la prédiction si l'individu cible possède ou non le caractère cible sur la base de valeurs des SNP de l'individu cible dans les un ou plusieurs loci génétiques identifiés.

2. Procédé mis en oeuvre par ordinateur selon la revendication 1, dans lequel le caractère cible est un caractère composite qui est défini au moins partiellement par une combinaison d'une pluralité de réponses d'enquête.

3. Procédé mis en oeuvre par ordinateur selon la revendication 2, dans lequel le caractère composite est défini par une première réponse à une première question d'enquête relative à un type de caractère de la pluralité d'individus et une deuxième réponse à une deuxième question d'enquête relative au même type de caractère de parents ou de grands-parents de la pluralité d'individus.

4. Procédé mis en oeuvre par ordinateur selon la revendication 3, dans lequel le caractère composite est défini par le fait que la première réponse est identique à la deuxième réponse.

5. Procédé mis en oeuvre par ordinateur selon la revendication 2, dans lequel le caractère composite est défini au moins par une réponse d'enquête et une autre valeur de données qui n'est pas déduite de réponses d'enquête.

6. Procédé mis en oeuvre par ordinateur selon la revendication 1, dans lequel l'analyse d'enrichissement comporte un test exact de Fisher.

7. Procédé mis en oeuvre par ordinateur selon la revendication 1, dans lequel l'étude d'association est une étude d'association pangénomique (GWAS).

8. Procédé mis en oeuvre par ordinateur selon la revendication 1, dans lequel la pluralité de communautés génétiques sont identifiées sur la base de liaisons d'identités par filiation (IBD) parmi les individus.

9. Procédé mis en oeuvre par ordinateur selon la revendication 1, dans lequel la pluralité de communautés génétiques sont identifiés sur la base d'un modèle de Markov caché qui divise les valeurs de SNP de chaque individu en une pluralité de fenêtres, chaque fenêtre comprenant une ou plusieurs étiquettes d'ethnicité.

10. Procédé mis en oeuvre par ordinateur selon la revendication 1, dans lequel au moins certaines des métadonnées sont déduites de réponses d'enquête comprenant des réponses à une ou plusieurs questions d'enquête, les une ou plusieurs questions d'enquête comprenant une ou plusieurs de ce qui suit : une question de caractère d'apparence, une question socio-économique, une question culturelle, une question de préférence, une question géographique, une question liée à la santé et une question d'antécédents familiaux.

11. Support non transitoire lisible par ordinateur configuré pour stocker des instructions qui, lorsqu'elles sont exécutées par un ou plusieurs processeurs, amènent les un ou plusieurs processeurs à réaliser un procédé comprenant des étapes selon l'une quelconque des revendications 1 à 10.

12. Système comprenant :
un ou plusieurs processeurs ; et
un support de stockage configuré pour stocker des instructions qui, lorsqu'elles sont exécutées par les un ou plusieurs processeurs, amènent les un ou plusieurs processeurs à réaliser un procédé comprenant des étapes selon l'une quelconque des revendications 1 à 10.
